# EUROPEAN PATENT SPECIFICATION

(11) **EP 2 964 675 B1**
(45) Date of publication and mention of the grant of the patent: **20.06.2018**
(21) Application number: 14714473.7
(22) Date of filing: 05.03.2014
(51) Int. Cl.: C07K 16/28, A61K 35/14, C07K 16/46, A61K 35/17, A61N 5/10, A61K 39/00, A61K 39/395, A61K 45/06, C07K 16/30, C07K 16/40

(54) **ENGAGER CELLS FOR IMMUNOTHERAPY**
ENGAGERZELLEN FÜR DIE IMMUNTHERAPIE
CELLULES D'ENGAGEMENT POUR IMMUNOTHÉRAPIE

(30) Priority: 05.03.2013 US 201361772803 P; 09.03.2013 US 201361775524 P; 16.01.2014 US 201461928383 P; 19.02.2014 US 201461941729 P
(43) Date of publication of application: 13.01.2016
(60) Divisional application: 18171595.4
(73) Proprietor: Baylor College Of Medicine, Houston, TX 77030 (US); Celgene Corporation, Summit, NJ 07901 (US)
(72) Inventor: GOTTSCHALK, Stephen, M. G., Houston, TX 77030 (US); SONG, Xiao-Tong, Pearland, TX 77584 (US); IWAHORI, Kota, Houston, TX 77030 (US); VELASQUEZ, Mireya, Paulina, Houston, TX 77030 (US); ABBOTT, Sterwart, Warren, NJ 07059 (US)
(74) Representative: J A Kemp
(86) International application number: PCT/US2014/020919
(87) International publication number: WO 2014/138306

(56) References cited:
- WO-A2-2009/091826
- US-A1- 2013 071 414
- BIPULENDU JENA ET AL: "Chimeric Antigen Receptor (CAR)-Specific Monoclonal Antibody to Detect CD19-Specific T Cells in Clinical Trials", PLOS ONE, vol. 8, no. 3, 1 March 2013 (2013-03-01), page e57838, XP055122892, DOI: 10.1371/journal.pone.0057838
- HAMMOND SCOTT A ET AL: "Selective targeting and potent control of tumor growth using an EphA2/CD3-bispecific single-chain antibody construct", CANCER RESEARCH, AMERICAN ASSOCIATION FOR CANCER RESEARCH, US, vol. 67, no. 8, 1 April 2007 (2007-04-01), pages 3927-3935, XP002578205, ISSN: 0008-5472
- LOEFFLER A ET AL: "Efficient elimination of chronic lymphocytic leukaemia B cells by autologous T cells with a bispecific anti-CD19/anti-CD3 single-chain antibody construct", LEUKEMIA, MACMILLAN PRESS LTD, US, vol. 17, no. 5, 1 May 2003 (2003-05-01), pages 900-909, XP002299902, ISSN: 0887-6924, DOI: 10.1038/SJ.LEU.2402890
- DIRK NAGORSEN ET AL: "Immunomodulatory therapy of cancer with T cell-engaging BiTE antibody blinatumomab", EXPERIMENTAL CELL RESEARCH, ACADEMIC PRESS, US, vol. 317, no. 9, 10 March 2011 (2011-03-10), pages 1255-1260, XP028205663, ISSN: 0014-4827, DOI: 10.1016/J.YEXCR.2011.03.010 [retrieved on 2011-03-16]
- DIRK NAGORSEN ET AL: "Immunotherapy of lymphoma and leukemia with T-cell engaging BiTE antibody blinatumomab", LEUKEMIA AND LYMPHOMA, INFORMA HEALTHCARE, US, vol. 50, no. 6, 1 January 2009 (2009-01-01), pages 886-891, XP008128752, ISSN: 1042-8194, DOI: 10.1080/10428190902943077
- DONALD B KOHN ET AL: "CARs on Track in the Clinic", MOLECULAR THERAPY, vol. 19, no. 3, 1 March 2011 (2011-03-01), pages 432-438, XP55122212, ISSN: 1525-0016, DOI: 10.1038/mt.2011.1
- BAEUERLE PATRICK A ET AL: "BiTE: Teaching antibodies to engage T-cells for cancer therapy", CURRENT OPINION IN MOLECULAR THERAPEUTICS, THOMSON REUTERS (SCIETIFIC) LTD, vol. 11, no. 1, 1 February 2009 (2009-02-01), pages 22-30, XP009151509, ISSN: 2040-3445
- COLE CAROLINE ET AL: "Tumor-targeted, systemic delivery of therapeutic viral vectors using hitchhiking on antigen-specific T cells", NATURE MEDICINE, NATURE PUBLISHING GROUP, NEW YORK, NY, US, vol. 11, no. 10, 1 October 2005 (2005-10-01), pages 1073-1081, XP002470982, ISSN: 1078-8956, DOI: 10.1038/NM1297
- HEGDE MEENAKSHI ET AL: "Combinational targeting offsets antigen escape and enhances effector functions of adoptively transferred T cells in glioblastoma.", MOLECULAR THERAPY : THE JOURNAL OF THE AMERICAN SOCIETY OF GENE THERAPY NOV 2013, vol. 21, no. 11, November 2013 (2013-11), pages 2087-2101, XP002725708, ISSN: 1525-0024
- FENG YU ET AL: "T-cell Engager-armed Oncolytic Vaccinia Virus Significantly Enhances Antitumor Therapy", MOLECULAR THERAPY, vol. 22, no. 1, 17 October 2013 (2013-10-17), pages 102-111, XP055122707, ISSN: 1525-0016, DOI: 10.1038/mt.2013.240

## Description

### TECHNICAL FIELD

The fields of the invention include at least immunology, cell biology, molecular biology, and medicine, including oncology.

### BACKGROUND

Immunotherapy with antigen-specific T-cells has shown promise in the treatment of solid tumors and hematological malignancies in preclinical models as well as in Phase I/II clinical studies. Genetically modifying T-cells with chimeric antigen receptors (CARs) or engineered T-cell receptors (TCRs) allows for the rapid generation of antigen-specific T-cells. However, neither CAR nor engineered TCR expressing T-cells redirect the vast reservoir of resident immune cells to tumors limiting anti-tumor effects.

To overcome this limitation, a new approach to render immune cells not only specific for tumor cells but also to allow them to redirect resident immune cells to tumor sites is described herein. The subject matter of this disclosure has applications not only to the field of cancer immunotherapy, but to any other disease in which the immune system is manipulated for therapeutic purposes.

WO 2009/091826 discloses compositions and methods related to a human CD19-specific chimeric T cell receptor polypeptide comprising and intracellular signaling domain, a transmembrane domain and an extracellular domain, the extracellular domain comprising a human CD19 binding region.

### SUMMARY

In a first aspect, provided herein is a composition comprising, or consisting essentially of, or consisting of, an engager, wherein said engager is a molecule that comprises an activation domain that binds to an activation molecule on an immune cell surface or an engineered immune cell surface, and an antigen recognition domain that binds to a target cell antigen, e.g., an antigen expressed on the surface of a tumor cell or cancer cell. The cancer cell may be of a solid tumor or a hematological malignancy.

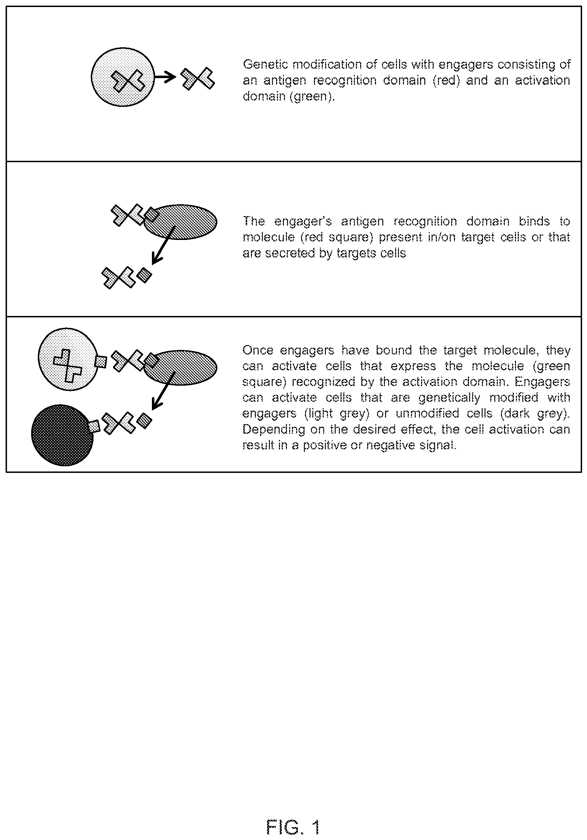

Engager cells are cells that have the capability of secreting engager molecules (FIG. 1), and in certain aspects of the invention, the individual is provided with cells that provide therapy to the individual. The cells (including T-cells and NK cells) secrete engagers that activate immune cells.

In certain aspects described herein, when the activation domain binds to the activation molecule on the immune cell, and the antigen recognition domain binds to the target cell antigen, the immune cell kills the target cell. The engager may be bipartite (*e.g*., comprising an activation domain and antigen recognition domain that may optionally be joined by a linker), or may be tripartite or multipartite (*e.g*., comprise one or more activation domains and/or antigen recognition domains, or other domains).

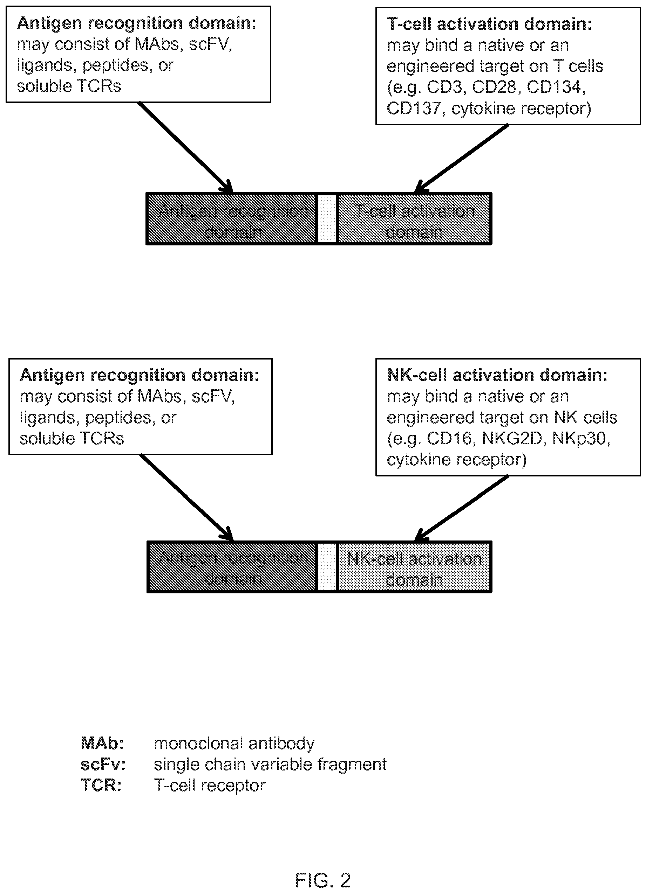

In certain embodiments, the engager is a protein, *e.g.,* an engineered protein. In specific aspects described herein, the activation domain of the engager is or comprises an antibody or an antigen-binding fragment or portion thereof, *e.g.,* a single chain variable fragment (scFv). In other specific aspects described herein, the antigen recognition domain is or comprises an antibody or an antigen-binding fragment or portion thereof, *e.g.,* a monoclonal antibody or an scFv, or it may comprise ligands, peptides, soluble T-cell receptors, or combinations thereof (**FIG. 2**). In certain embodiments, the activation domain and antigen recognition domain are joined by a linker, *e.g.,* a peptide linker.

The activation domain of an engager molecule can provide activation to immune cells. The skilled artisan recognizes that immune cells have different activating receptors. For example CD3 is an activating receptor on T-cells, whereas CD16, NKG2D, or NKp30 are activating receptors on NK cells, and CD3 or an invariant TCR are the activating receptors on NKT-cells. Engager molecules that activate T-cells may therefore have a different activation domain than engager molecules that activate NK cells (**FIG. 2**). In specific aspects described herein, *e.g*., wherein the immune cell is a T-cell, the activation molecule is one or more of CD3, *e.g*., CD3γ, CD3δ or CD3ε; or CD27, CD28, CD40, CD134, CD137, and CD278. In other specific aspects described herein, *e.g*., wherein the immune cell is a NK cell, the activation molecule is CD16, NKG2D, or NKp30, or wherein the immune cell is a NKT-cell, the activation molecule is CD3 or an invariant TCR, or wherein the immune cell is a NKT cell, the activation molecule is an invariant TCR is CD1d.

Activation can either result in a positive or a negative signal. Examples for 'positive signal' activation domains include 1) scFvs that are specific for (and activate) CD3 and ligands or receptors of co-stimulatory molecules, such as CD28, CD134, and CD137; 2) domains derived from co-stimulatory molecules, such as CD80, CD70, CD134L (OX40L), and CD137L (41BBL); 3) cytokines, such as IL-2, IL-7, and IL-15; and 4) chemokines such as CCL1, CCL2, CCL 16, CCL 17, CCL 22, CXCL8, or RANTES. Examples for 'negative signal' activation domains include 1) domains derived from inhibitory molecules, such as PD-L1; 2) scFvs specific for inhibitory molecules such as CTLA4 and PD-1, and 2) inhibitory cytokines, such as TGFβ and IL-10. In a specific embodiment, the activation domain is an scFv.

In certain other embodiments, the engager additionally comprises one or more accessory domains, *e.g*., one or more of a cytokine, a costimulatory domain, a domain that inhibits negative regulatory molecules of T-cell activation, or a combination thereof. In specific embodiments, the cytokine is IL-15, IL-2, and/or IL-7. In other specific embodiments, the costimulatory domain is CD27, CD80, CD86, CD134, or CD137. In other specific embodiments, the domain that inhibits negative regulatory molecules of T-cell activation is PD-1, PD-L1, CTLA4, or B7-H4.

For any of the engagers described herein, the respective domains may be in any order N-terminus to C-terminus, including, *e.g*., having the activation domain N-terminal to the antigen recognition domain, having the activation domain C-terminal to the antigen recognition domain, and so forth. In certain embodiments, T-cells are modified to secrete engager molecules that have the antigen recognition domain N-terminal to the activation domain. In particular embodiments, two or more of the domains of an engager molecule are separated by a linker. The linker may be of any suitable length, and such a parameter is routinely optimized in the art. For example, linkers may be of a length and sequence sufficient to ensure that each of the first and second domains can, independently from one another, retain their differential binding specificities.

In certain embodiments, the antigen bound by the antigen recognition domain is a tumor-associated antigen (TAA) or a tumor-specific antigen (TSA). In certain embodiments, the antigen recognition domain of the engager is an scFv that is specific for a TAA or TSA. In a specific embodiment, the TAA or TSA is expressed on a cancer cell. In one embodiment, the TAA or TSA is expressed on a blood cancer cell. In another embodiment, the TAA or TSA is expressed on a cell of a solid tumor. In more specific embodiments, the solid tumor is a glioblastoma, a non-small cell lung cancer, a lung cancer other than a non-small cell lung cancer, breast cancer, prostate cancer, pancreatic cancer, liver cancer, colon cancer, stomach cancer, a cancer of the spleen, skin cancer, a brain cancer other than a glioblastoma, a kidney cancer, a thyroid cancer, or the like. In more specific embodiments, the TAA or TSA is expressed by a tumor cell in an individual. In specific aspects described herein, the TAA or TSA is one or more of, *e.g.,* an scFv on the engager is specific for one or more of 5T4, 8H9, αᵥβ₆ integrin, BCMA, B7-H3, B7-H6, CAIX, CA9, CD19, CD20, CD22, CD30, CD33, CD38, CD44, CD44v6, CD44v7/8, CD70, CD123, CD138, CD171, CEA, CSPG4, EGFR, EGFR family including ErbB2 (HER2), EGFRvIII, EGP2, EGP40, , ERBB3, ERBB4, ErbB3/4, EPCAM, EphA2, EpCAM, FAP, FBP, fetal AchR, FRα, GD2, GD3, Glypican-3 (GPC3), HLA-A1+MAGE1, HLA-A1+NY-ESO-1, IL-11Rα, IL-13Rα2, Lambda, Lewis-Y, Kappa, KDR, MCSP, Mesothelin, Muc1, Muc16, NCAM, NKG2D Ligands, NY-ESO-1, PRAME, PSC1, PSCA, PSMA, ROR1, SP17, Survivin, TAG72, TEMs, carcinoembryonic antigen, HMW-MAA, and VEGFR2.

The particular cell recognition domain of the engager molecule may be tailored to recognize a corresponding tumor expressing a particular antigen. Antigens may be either produced by a malignant cancer cell or by the so-called tumor stroma, which support tumor growth. In some instances, the scFv is specific for EphA2, CD19, CD123, LeY, B7H3, HER2, or EGFR (including EGFRVIII). Exemplary antigens are listed in **Table 1.**

**Table 1: Examples of targetable antigens**

| Antigen |
|---|
| 5T4 |
| αᵥβ₆ integrin |
| B7-H3 |
| B7-H6 |
| CD19 |
| CD20 |
| CD22 |
| CD30 |
| CD33 |
| CD44, CD44v6, CD44v7/8 |
| CD70 |
| CD123 |
| CEA |
| CSPG4 |
| EGFR, EGFR family including ErbB2 (HER2) |
| EGFRvIII |
| EGP2 |
| EGP40 |
| EpCAM |
| EphA2 |
| FAP |
| fetal AchR |
| FRα |
| GD2 |
| GD3 |
| Glypican-3 (GPC3) |
| HLA-A1+MAGE1 |
| HLA-A2+MAGE1 |
| HLA-A3+MAGE1 |
| HLA-A1+NY-ESO-1 |
| HLA-A2+NY-ESO-1 |
| HLA-A3+NY-ESO-1 |
| IL-11Rα |
| IL-13Rα2 |
| Lambda |
| Lewis-Y |
| Kappa |
| Mesothelin |
| Muc1 |
| Muc1 6 |
| NCAM |
| NKG2D Ligands |
| NY-ESO-1 |
| PRAME |
| PSCA |
| PSMA |
| ROR1 |
| TAG72 |
| TEMs |
| VEGFR2 |

Besides the examples of antigens listed in **Table 1,** the antigen recognition domain can also target other antigens; for example inhibitory molecules expressed on cancer cells or cells within the tumor microenvironment, such as PD-L1 and CTLA4. An engager molecule that comprises a PD-L1-specific antigen recognition domain and a CD3-specific T-cell activation domain would overcome tumor-mediated immunosuppression by converting an inhibitory signal into a positive (CD3-activating) signal. Other examples are antigens that are present with in the extracelluar matrix of tumors such as oncofetal variants of fibronectin, tenascin, or necrotic regions of tumors.

In another aspect, provided herein is a polynucleotide that encodes an engager molecule, e.g., any of the engager molecules described herein. The polynucleotide may be comprised within, or comprise, a vector. Any type of suitable vector may be employed, but in specific embodiments the vector is a non-viral vector (*e.g.,* a plasmid, a minicircle DNA vector, a sleeping beauty plasmid, a piggybac plasmid, and so forth) or a viral vector (*e.g.,* a lentiviral vector, adenoviral vector, retroviral vector, adeno-associated viral vector, oncolytic vector, and so forth). In a specific embodiment, the polynucleotide stably expresses the engager molecule in a cell that contains the polynucleotide. In another specific embodiment, the polynucleotide transiently expresses the engager molecule in a cell that contains the polynucleotide. In another specific embodiment, the polynucleotide allows inducible expression of the engager molecule in a cell that contains the polynucleotide. In another specific embodiment, the polynucleotide allows inducible repression of expression of the engager molecule in a cell that contains the polynucleotide. In any of the embodiments provided herein, the polynucleotide preferably encodes the engager in a form that is secretable by the cell. The polynucleotide encodes a fusion molecule comprising an activation domain and an antigen recognition domain, and in specific embodiments each domain is a scFv. The activation domain may be positioned toward the N-terminus of the polypeptide in relation to the antigen recognition domain, or the activation domain may be positioned toward the C-terminus of the polypeptide in relation to the antigen recognition domain.

In another aspect, provided herein is a cell that has been genetically engineered to express one or more engagers, *e.g*., any of the engager molecules described herein (*e.g.,* referred to as an "engager cell"). In certain aspects described herein, the genetically engineered cell is, *e.g.,* a T lymphocyte (T-cell), a CAR T-cell, a natural killer (NK) T-cell, or an NK cell. In certain other aspects described herein, the genetically engineered cell is a non-immune cell, *e.g.,* a mesenchymal stem cell (MSC), a neuronal stem cell, a hematopoietic stem cell, an induced pluripotent stem cell (iPS cell), an embryonic stem cell. In certain embodiments, the engager cell secretes the engager into the environment surrounding the engager cell, *e.g.,* culture medium (where the engager cell is cultured *in vitro*) or into an individual into which the engager cell has been administered). Although in certain embodiments a lentiviral vector may be employed, in some cases a retroviral vector having higher transduction efficiency may be employed. An exemplary retroviral vector comprises in a 5' to 3' direction CD19scFv-CD3scFv. Another exemplary retroviral vector comprises in a 5' to 3' direction EphA2(4H5)-CD3scFv, optionally followed by IRES followed by a nucleotide sequence encoding a fluorescent or selectable marker, *e.g*., mOrange.

In certain embodiments, a cell secretes more than one (*e.g.,* 1, 2, 3, 4, 5, 6, 7, 8, 9, 10 or more) type of engager molecule. The cell may be modified to harbor non-identical polynucleotides that express non-identical engager molecules. For example, one cell may comprise one polynucleotide that encodes an engager molecule and also comprise one or more other polynucleotides that encode other engager molecules. In specific embodiments, the cell harbors a first polynucleotide that encodes an engager molecule that targets one of the antigens listed in **Table 1** and a second polynucleotide that encodes an engager molecule that targets another antigen listed in **Table 1.** Besides different antigen recognition domains, the engagers described herein may also contain different activation domains so that immune cells can activate T-cells or NK cells, or both. Thus, aspects of the disclosure provide for T-cells that for example secrete engager molecules that can activate NK cells and/or T-cells, and NK cells that activate NK and/or T-cells.

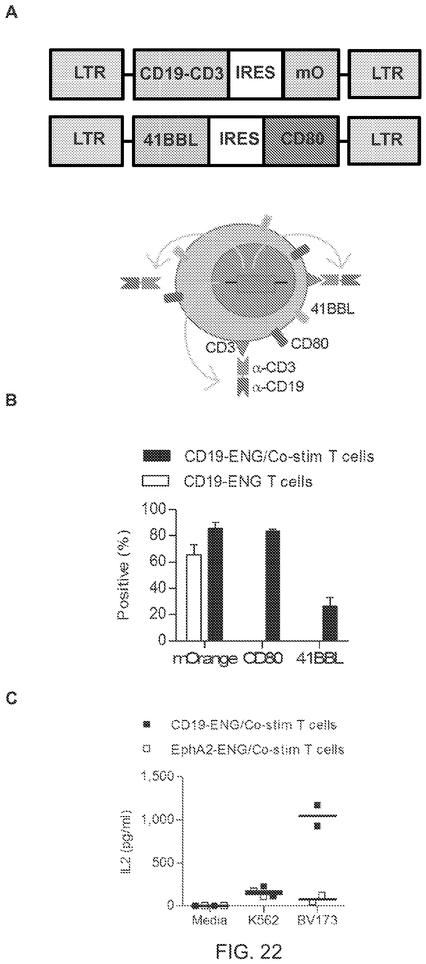

Although in specific embodiments the engager cells of the present invention do not also comprise a CAR, an engineered TCR, or any other genetic modification, in certain embodiments the engager T-cells also comprise a CAR, including one that targets antigens listed in **Table 1,** an engineered TCR, or any other genetic modification that may enhance its function. In one embodiment, a CAR or engineered TCR expressing T-cell comprises one or more engager molecules. In a particular embodiment, an antigen binding domain of a CAR or TCR and an antigen recognition domain of an engager molecule recognize or bind to the same target antigen. In a certain embodiment, an antigen binding domain of a CAR and an antigen recognition domain of an engager molecule recognize or bind to different target antigens expressed on the same target cell. In a specific embodiment, a cell comprises one polynucleotide that expresses an engager molecule and also comprises one or more polynucleotides that express co-stimulatory molecules, including CD80, CD70, CD134L (OX40L), and CD137L (41BBL). In specific embodiments, the cell harbors a first polynucleotide that encodes an engager molecule that targets CD19 and a second polynucleotide that encodes CD80 and 41BBL (**FIG. 22**).

In particular aspects described herein, there are pharmaceutical compositions that include an engager molecule and/or one or more cells that secrete an engager molecule, including one that has an activation domain that binds to a target on a native immune cell surface or an engineered immune cell surface and also a recognition domain that binds one or more molecules produced by a target cell. An effective amount of the engager molecules or cells that secrete them are given to an individual in need thereof.

In another aspect, provided herein is a method of treating an individual having a tumor cell, comprising administering to the individual a therapeutically effective amount of an engager molecule, wherein the engager molecule comprises an antigen recognition domain that binds to an antigen on the tumor cell. In a related aspect, provided herein is a method of treating an individual having a tumor cell, comprising administering to the individual a therapeutically effective amount of engager cells that secrete one or more engager molecules, wherein the engager molecules comprise an antigen recognition domain that binds to an antigen on the tumor cell. In a specific aspects described herein, said administering results in a measurable decrease in the growth of the tumor in the individual. In another specific aspects described herein, said administering results in a measurable decrease in the size of the tumor in the individual. In various aspects described herein, the size or growth rate of a tumor may be determinable by, *e.g.,* direct imaging (*e.g.,* CT scan, MRI, PET scan or the like), fluorescent imaging, tissue biopsy, and/or evaluation of relevant physiological markers (*e.g*., PSA levels for prostate cancer; HCG levels for choriocarcinoma, and the like). In specific aspects described herein, the individual has a high level of an antigen, *e.g*., EphA2, that is correlated to poor prognosis. In some aspects described herein, the individual is provided with an additional cancer therapy, such as surgery, radiation, chemotherapy, hormone therapy, immunotherapy, or a combination thereof.

In particular aspects described herein, there is a cell that secretes a composition, said composition comprising: an activation domain that binds to a target on a native immune cell surface or an engineered immune cell surface; and an antigen recognition domain that binds one or more molecules produced by a target cell. The activation domain and the antigen recognition domain are attached by a linker, in at least some cases. The activation domain may comprise an antibody or a ligand. In specific aspects described herein, the immune cell is a T-cell and the antibody recognizes CD3, although the immune cell may be a NK cell and the antibody recognizes CD16, NKG2D, or NKp30. In particular aspects described herein, the antibody is a single chain fragment variable (scFv) antibody. In specific aspects described herein, the antigen recognition domain is an antibody that recognizes an antigen produced by target cells, and in some cases the antibody is an scFv fragment.

In particular aspects described herein, the antigen recognition domain is a ligand, a peptide, a soluble TCR, or recognizes an antigen of Table 1.

Particular exemplary compositions of the disclosure comprise the sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, or SEQ ID NO:9, for example.

Some compositions of the disclosure further comprise one or more of the following: a cytokine; a co-stimulatory domain; or a domain for inhibition of negative regulatory molecules of T-cell activation. In specific aspects described herein, the cytokine is IL-15. In certain aspects, the co-stimulatory domain is CD80, CD137, or both. In particular aspects, the domain for inhibition of negative regulatory molecules of T-cell activation comprises PD-1, PD-L1, CTLA4, or B7-H4. Some compositions of the disclosure comprise a detectable marker.

In one aspect described herein, there is a method of treating an individual with cancer, comprising the step of delivering a therapeutically effective amount of one or more cells of the disclosure to the individual. In specific aspects described herein, the composition is secreted by the cells and the composition binds to and activates the cells. In certain aspects, the composition is secreted by the cells and the composition binds to and activates native immune cells in the individual. In particular aspects described herein, the cells that are capable of expressing the composition are T-cells and the activation domain comprises an antibody that recognizes CD3. In other aspects, the cells that are capable of expressing the composition are NK cells and the activation domain comprises an antibody that recognizes CD16, NKG2D, or NKp30. In one aspect, the cancer is EphA2-positive, CD19-positive, CD123-positive, LeY-positive, B7H3-positive, HER2-positive, or EGFR (including EGFRvIII)-positive.

In aspects described herein, there is a composition comprising a cell that secretes a polypeptide, said polypeptide comprising: an activation domain that binds to a target on an immune cell surface; and an antigen recognition domain that binds one or more molecules produced by or present on a target cell. In specific aspects described herein, the activation domain and the antigen recognition domain are attached by a linker. In specific embodiments, the immune cell is a native immune cell. In certain aspects described herein, the immune cell is an engineered immune cell. In particular embodiments, the activation domain comprises an antibody, ligand, receptor, or peptide. In particular aspects described herein, the immune cell is a T-cell and the antibody recognizes CD3. In some aspects described herein, the immune cell is a NK cell and the antibody recognizes CD16, NKG2D, or NKp30. In certain aspects described herein, the antibody is a single chain fragment variable (scFv) antibody. In particular aspects described herein, the antigen recognition domain is an antibody that recognizes an antigen produced by target cells. In some aspects described herein, the antibody is a scFv antibody. In particular aspects described herein, the antigen recognition domain is a ligand, a peptide, or a soluble TCR. In specific aspects described herein, the antigen recognition domain recognizes an antigen of Table 1. In particular aspects described herein, the polypeptide comprises the sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8, or SEQ ID NO:9. In certain aspects described herein, the polypeptide further comprises one or more of the following: a cytokine; a co-stimulatory domain; or a domain for inhibition of negative regulatory molecules of T-cell activation. In specific aspects described herein, the cytokine is IL-15, IL-12, IL-2, or IL-7. In specific aspects described herein, the co-stimulatory domain is CD80, CD134, CD137, or a combination thereof. In certain aspects described herein, the domain for inhibition of negative regulatory molecules of T-cell activation comprises PD-1, PD-L1, CTLA4, or B7-H4. In some aspects described herein, the polypeptide further comprises a detectable marker. In some aspects described herein, the cell comprises a polynucleotide encoding the polypeptide. In certain aspects described herein, the polynucleotide is integrated into the genome of the cell. In particular aspects described herein, expression of the polynucleotide encoding the polypeptide is under the control of one or more tumor-associated factors. In some aspects described herein, the one or more tumor-associated factors is a chemokine or cytokine. Chemokines may be from a family selected from the group consisting of CC, CXC, C, and CX3C chemokines, including CCL2, CCL3, CCL5, CCL16, CXCL8, CXCL10, XCL1, XCL2, or CX3CL1. Cytokines may be selected from the group consisting of IL2, IL4, IL5, IL7, IL10, IL12, IL15, and IL17. In specific aspects described herein, expression of the polynucleotide encoding the polypeptide is under the control of a tissue-specific regulatory sequence. In some aspects described herein, the tissue-specific regulatory sequence is a hypoxia-specific regulatory sequence. In certain aspects described herein, the hypoxia-specific regulatory sequence is a hypoxia response element (HRE) or an oxygen-dependent degradation domain (ODDD). In some aspects, the HRE comprises 5'-RCGTG-3' (SEQ ID NO:10).

In one aspect described herein t, there is a method of treating an individual with cancer, comprising the step of delivering a therapeutically effective amount of a composition of the disclosure to the individual. In specific aspects described herein, the polypeptide is secreted by the cells and the composition binds to and activates the cells, and in at least some cases the polypeptide is secreted by the cells and the polypeptide binds to and activates native immune cells in the individual. In some aspects described herein, the cells that are capable of expressing the polypeptide are T-cells. In certain aspects described herein, the activation domain comprises an antibody that recognizes CD3. In some aspects described herein, the cells that are capable of expressing the polypeptide are NK cells. In specific aspects described herein, the activation domain comprises an antibody that recognizes CD16, NKG2D, or NKp30. In certain aspects described herein, the cancer is EphA2-positive, CD19-positive, CD123-positive, LeY-positive, B7H3-positive, HER2-positive, or EGFR-positive, and may be EGFRvIII-positive.

The present invention provides:
- A cell comprising a polynucleotide vector encoding a bipartite molecule comprising an activation domain that binds to one or more cell surface molecules and an antigen recognition domain that binds to EphA2 and/or CD19, wherein the activation domain is a single chain variable fragment (scFv) that recognizes CD3 and wherein the cell is a T cell or NK cell;
- A cell of the invention for use in method of treating an individual with cancer, the method comprising the step of delivering a therapeutically effective amount of the cells to the individual; and
- A polynucleotide vector encoding a bipartite molecule comprising an activation domain that binds to one or more cell surface molecules and an antigen recognition domain that binds to EphA2 and CD19, wherein the activation domain is a single chain variable fragment (scFv) that recognizes CD3.

### BRIEF DESCRIPTION OF THE DRAWINGS

For a more complete understanding of the present invention, reference is now made to the following descriptions taken in conjunction with the accompanying drawing, in which:
**FIG. 1** illustrates the concept of apects of the engager cells.
**FIG. 2** illustrates the concept of aspects of the T-cell and NK-cell engagers.

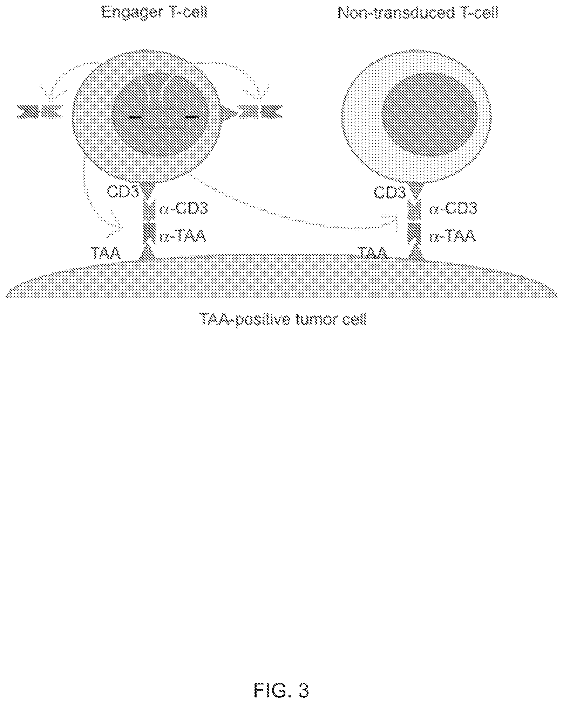

**FIG. 3** shows an exemplary Engager T-cell.

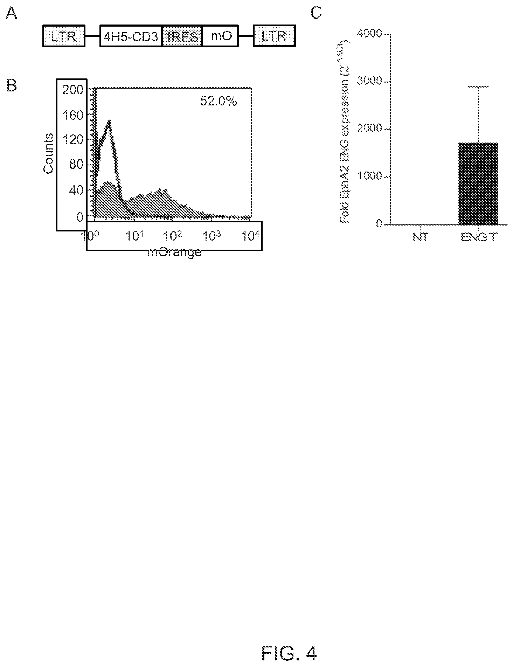

**FIG. 4** shows the generation of EphA2-ENG T-cells. (**A**) Scheme of retroviral vector (IRES: internal ribosomal entry site; mO: mOrange). (**B**) FACS analysis for mOrange of transduced and NT T-cells. (**C**) qRT-PCR for EphA2-engager of transduced and NT T-cells.

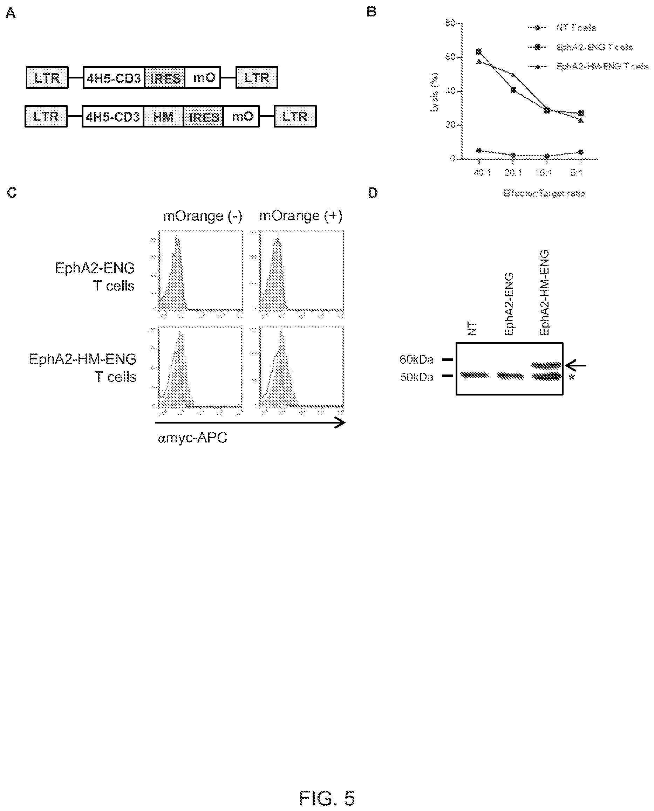

**FIG. 5** demonstrates that EphA2-specific engager molecules bind to the cells surface of T-cells and are secreted by T-cells. (**A**) Scheme of retroviral vectors encoding EphA2-ENG and EphA2-ENG with a c-terminal 6xHis-Myc tag (EphA2-HM). EphA2-HM was generated by inserting a 6xHisMyc-tag before the stop codon. EphA2-ENG and EphA2-HM ENG T-cells were generated by retroviral transduction and post transduction 73% (EphA2-ENG) or 57% (EphA2-HM ENG) T-cells were positive for mOrange by FACS analysis (data not shown). (**B**) Cytotoxicity assay using NT, EphA2-ENG and EphA2-HM T-cells as effectors and EphA2-positive U373 cells as target cells. There was no significant difference between EphA2- and EphA2-HM ENG T-cells, indicating that the tag does not interfere with the function of the engager molecule. (**C**) FACS analysis of EphA2- and EphA2-HM ENG T-cells gated on mOrange positive and mOrange negative cells (Open curve: isotype, Filled curve: αmyc-APC (Abcam, Cambridge, MA). EphA2-ENG T-cells showed no cell surface staining. EphA2-ENG-HM T-cells were stained with αmyc-APC. Transduced (mOrange+) and non-tranduced T-cells (mOrange-) were positive, indicating that transduced T-cells secrete engager molecules that bind to non-transduced T-cells. (**D**) Media from NT, EphA2-ENG or EphA2-HM ENG T-cells were incubated with His Mag Sepharose excel (GE Healthcare). Beads were washed and the bound fraction was eluted according to the manufacturer's instruction. The eluted fraction was separated by SDS-PAGE, and blotted with αMyc (Abcam) followed by a HRP-conjugated goat mouse IgG antibody (Santa Cruz Biotechnology); *: unspecific band.

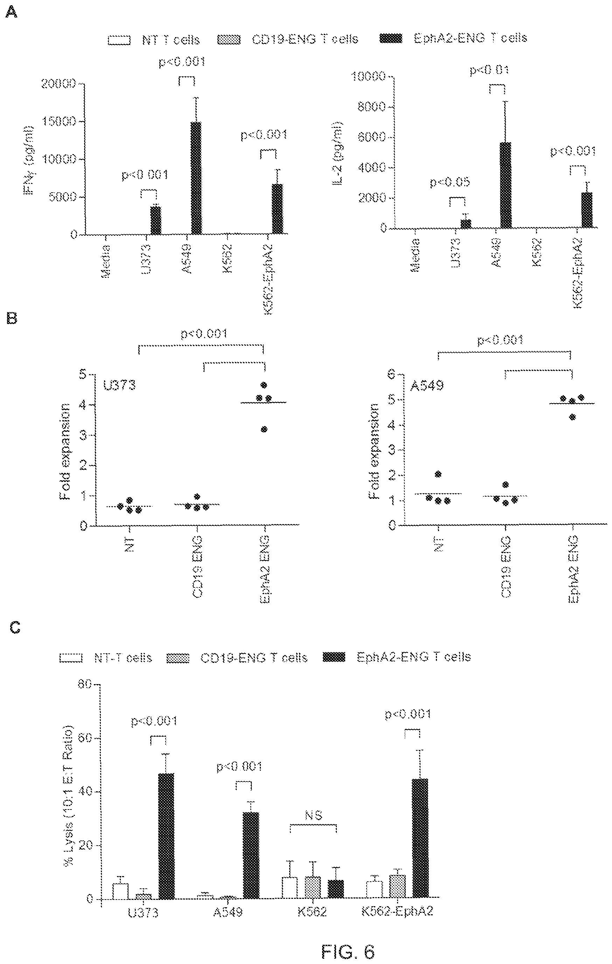

**FIG. 6** demonstrates that EphA2-ENG T-cells secrete cytokines, proliferate and kill target cells in an antigen-specific manner. (**A**) EphA2-ENG, CD19-ENG, and NT T-cells were cocultured with EphA2-positive (U373, A549, K562-EphA2) or -negative (K562) tumor cells at a ratio of 10:1. After 24 hours, IFNγ and IL2 production was determined by ELISA (n=4). (**B**) EphA2-ENG, CD19-ENG, and NT T-cells were cocultured with EphA2-positive (U373, A549) tumor cells at a ratio of 10:1. After 7 days, number of viable T-cells were enumerated by trypan blue staining (n=4). (**C**) Cytotoxicity assays were performed using EphA2-ENG, CD19-ENG, and NT T-cells as effectors and EphA2-positive (U373, A549, K562-EphA2) and -negative (K562) tumor cells as targets (mean ± SD; n=4).

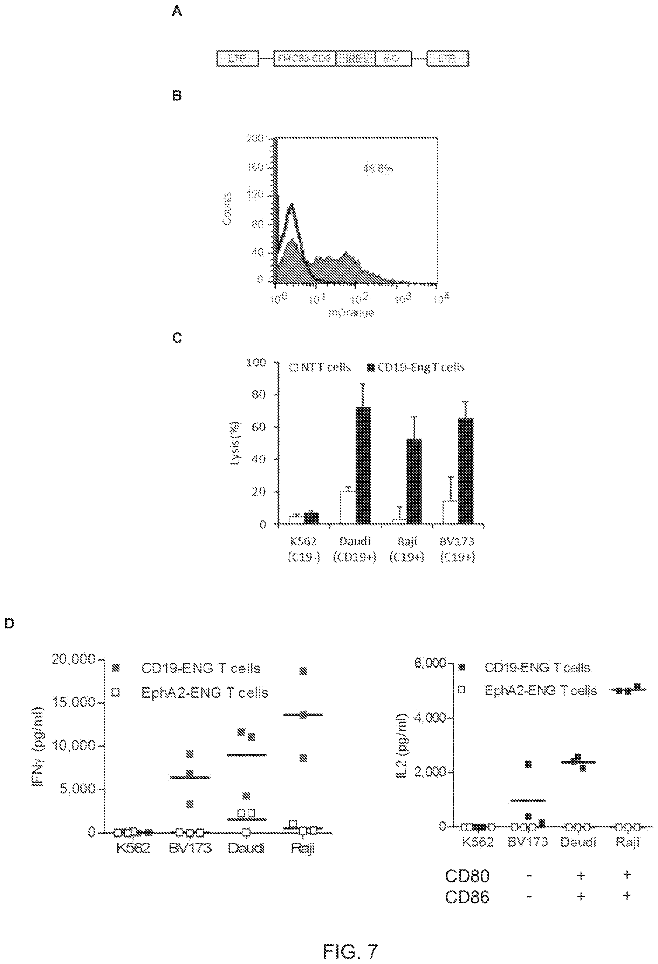

**FIG.** 7 demonstrates the generation of T-cells secreting CD19-specific engager molecules. (**A**) Scheme of retroviral vector. (**B**) FACS analysis for mOrange of transduced and NT T-cells. (**C**) In cytotoxicity assays only CD19-ENG T-cells killed CD19+ cell lines (Daudi, Raji, BV173) in contrast to non-transduced (NT) T-cells at a E:T ratio of 2.5:1 (n=4; p<0.05). (**D**) Only CD19-ENG T-cells secreted significant levels of IFN-γ in coculture assays with CD19+ targets (n=3; CD19-ENG vs EphA2-ENG T-cells for BV173, Daudi, Raji: p<0.05). Consistent IL-2 production was specific for CD19+ targets and ws influenced by expression of CD80 and CD86 on target cells (n=3; CD19-ENG vs EphA2-ENG T-cells for Daudi and Raji: p<0.05; CD19-ENG vs EphA2-ENG T-cells for BV173: p=NS).

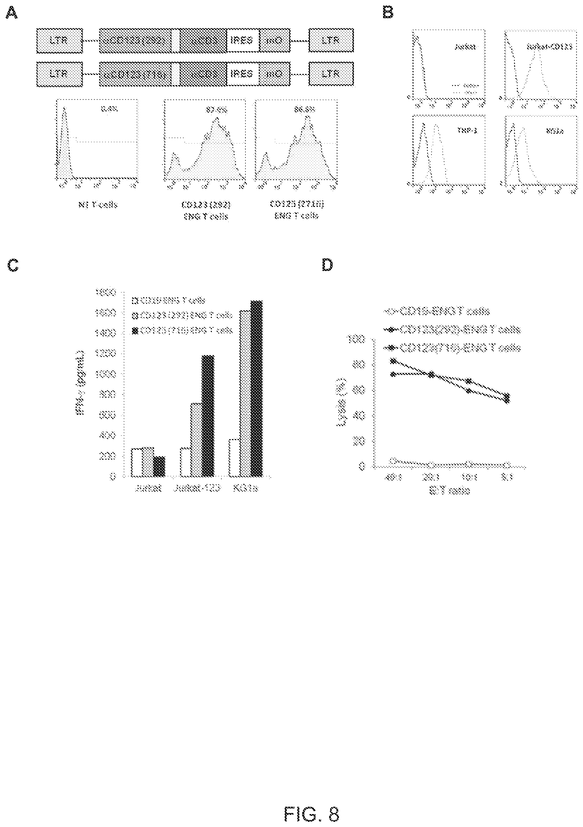

**FIG. 8** demonstrates that T-cells secreting CD123-specific T-cell engagers recognize CD123-positive tumor cells in an antigen-specific manner. (**A**) Scheme of retroviral vectors encoding CD123-specific Engagers and FACS analysis of transduced T-cells. (**B**) FACS analysis of CD123-positive AML cells (THP-1 and KG1a) and negative (Jurkat) and positive control (Jurkat CD123). (**C**) Coculture assay of CD19-, CD123(292)-, or CD123(716)-specific effectors with CD123-positive target cells (KG1a, Jurkat-CD123), and CD123-negative target cells (Jurkat). IPNγ was determined after 24h. (**D**) Cytotoxicity assay using CD19-, CD123(292)-, or CD123(716)-specific effectors and CD123-positive target cells (KG1a).

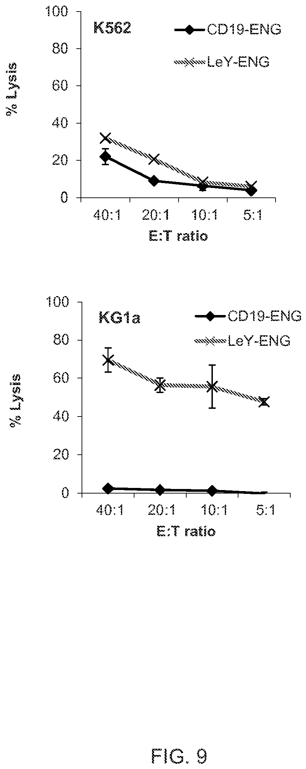

**FIG. 9** demonstrates that T-cells secreting LeY-specific T-cell engagers kill LeY-positive tumor cells in an antigen-specific manner. Standard cytotoxicity assay were performed with LeY-ENG T-cells and CD19-ENG T-cells as effectors and K562 (CD19-, LeY-) and KG1a (CD19-, LeY+) target cells.

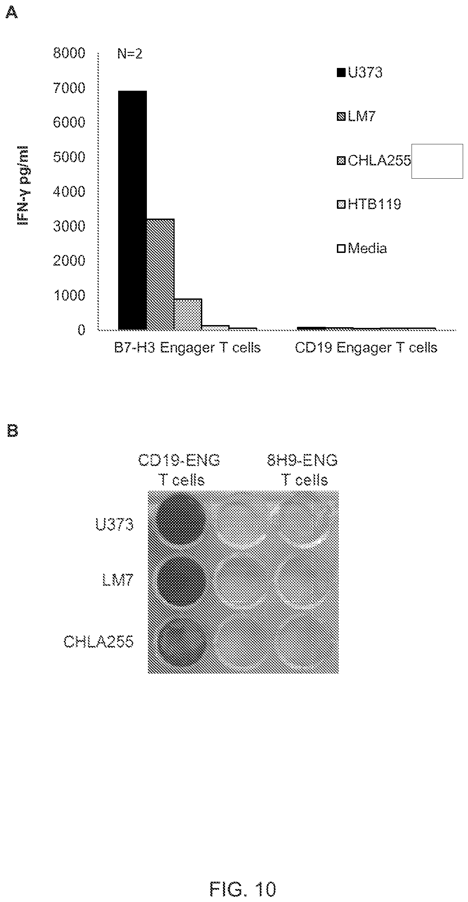

**FIG. 10** demonstrates that T-cells secreting B7H3-specific T-cell engagers recognize B7H3-positive tumor cells in an antigen-specific manner. (**A**) Co-culture assay of B7H3-ENG T-cells or CD19-ENG T-cells with B7H3-positive (U373, LM7, CHLA255) and B7H3-negative (HTB119) tumor cells. After 24 hours IPNγ was determined. (**B**) Co-culture assay of B7H3-ENG T-cells or CD19-ENG T-cells with U373, LM7, CHLA255. Viable tumor cells were visualized by crystal violet staining.

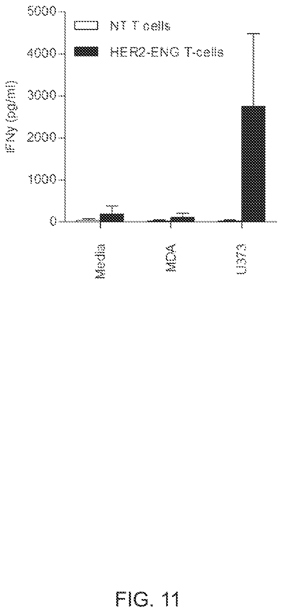

**FIG. 11** demonstrates that T-cells secreting HER2-specific T-cell engagers recognize HER2-positive tumor cells in an antigen-specific manner. HER2-ENG T-cells and non-transduced (NT) T-cells were co-cultured with HER2-positive (U373) and HER2-negative (MDA) tumor cells. After 24 hours IFNy was determined.

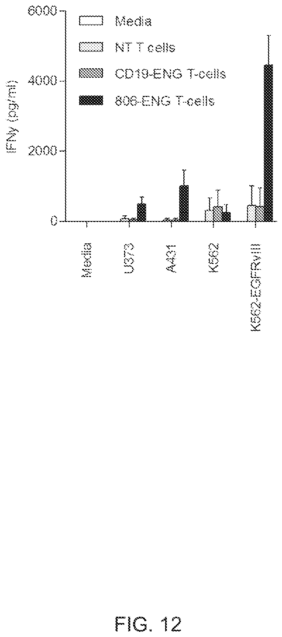

**FIG. 12** demonstrates that T-cells secreting T-cell engagers that are specific for the conformational EGFR epitope 806 recognize EGFR-positive tumor cells in an antigen-specific manner. 806-ENG T-cells and CD19-ENG T-cells were incubated with U373 (EGFR low positive), A431 (EGFR gene amplified), K562 (EGFR negative), and K562 genetically modified to express EGFRvIII (K562-EGFRvIII). After 24 hours IPNγ was determined.

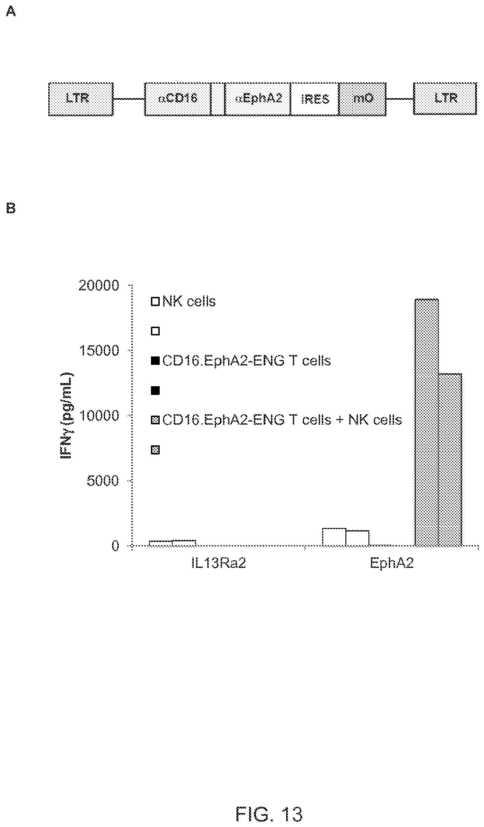

**FIG. 13** demonstrates that T-cells secreting an EphA2-specific NK-cell engagers activate NK cells in an antigen-specific manner. (**A**) Scheme of retroviral vectors encoding EphA2-specific NK-cell engager. (**B**) Coculture assay of NK cells, CD16.EphA2-ENG T-cells, or CD16.EphA2-ENG T-cells plus NK cells on IL13Rα2- or EphA2-coated plates. Only CD16.EphA2-ENG T-cells plus NK cells co-cultures in the presence of EphA2 produced high levels of IFNg, demonstrating antigen-specific activation of NK cells.

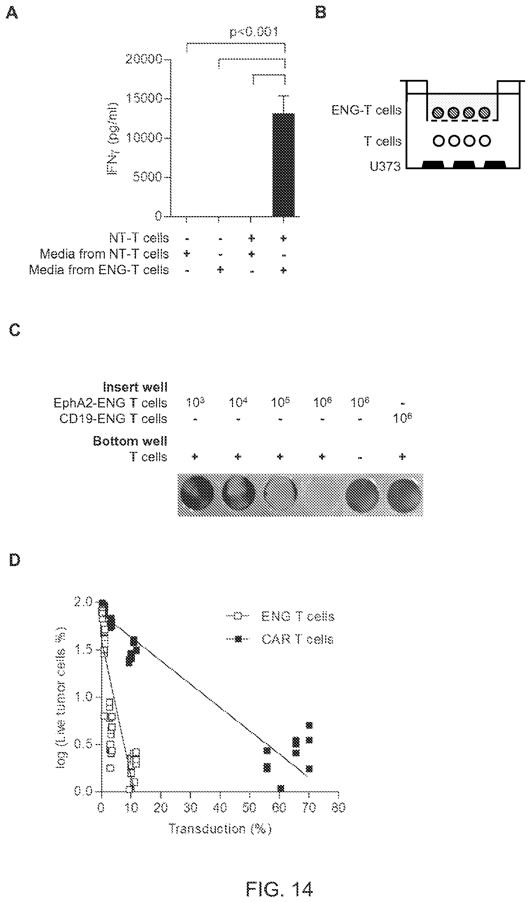

**FIG. 14** demonstrates that EphA2-ENG T-cells redirect bystander T-cells to tumor cells. (**A**) A549 cells were cocultured with or without NT T-cells and media of NT T-cells or EphA2-ENG T-cells. After 24 hours, IFN-γ production was determined by ELISA (mean ± SD; n=4). (**B**) Scheme of coculture transwell assays. (**C**) NT T-cells and U373 cells were plated in the bottom well, and EphA2-ENG T-cells in the insert well. The number of plated EphA2-ENG T-cells ranged from 10³ to 10⁶. CD19-ENG T-cells in the insert well and bottom wells without NT T-cells served as controls. After 48 hours, live U373 cells were visualized by crystal violet staining. Results of one representative experiment are shown. (**D**) To compare the antitumor activity of EphA2-ENG and EphA2-CAR T-cells U373 cells were incubated with 1x10⁵ T-cells containing increasing percentages of transduced EphA2-ENG or EphA2-CAR T-cells. After 48 hours viable tumor cells were measured by MTS assay (n=4; triplicate assay for each donor; p<0.00001).

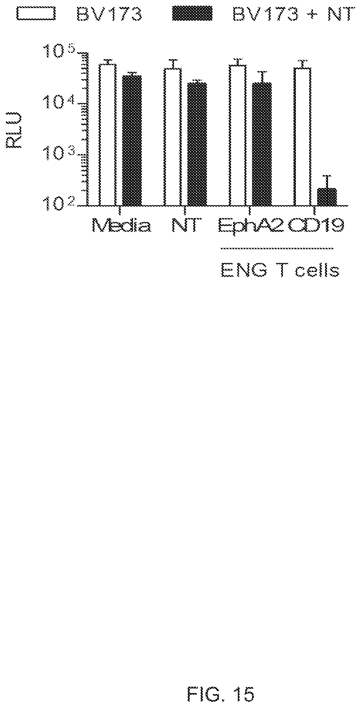

**FIG. 15** demonstrates that CD19-ENG T-cells redirect bystander T-cells to tumor cells. Non transduced (NT), EphA2-ENG, or CD19-ENG T-cells were plated in the insert well, and fire fly luciferase (ffLuc)-BV173 or ffLuc-BV173 and NT T-cells were plated in the bottom well. After 48h presence of tumor cells was determined by luc-assay. Only CD19-ENG T-cells redirected NT T-cells to tumor cells (n=3; P<0.05).

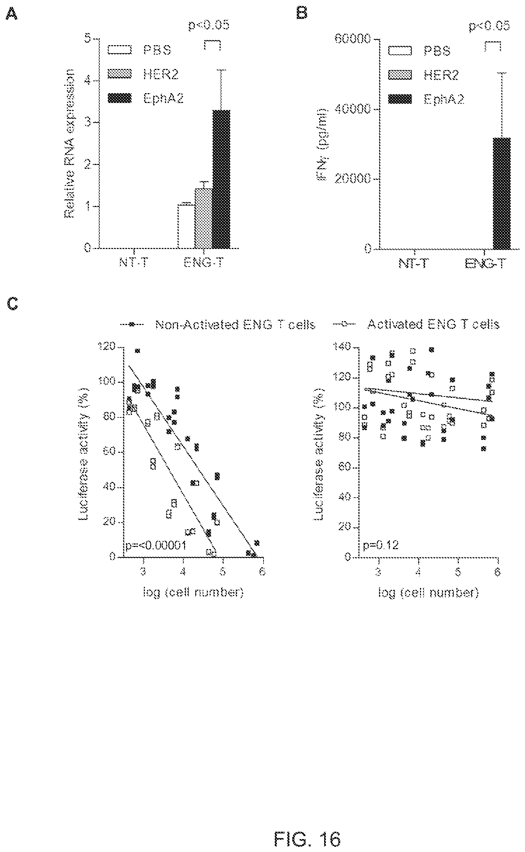

**FIG. 16** demonstrates that antigen-specific activation of EphA2-ENG T-cells enhances their ability to redirect bystander T-cells to tumor cells. (**A,B**) NT T-cells and EphA2-ENG T-cells were cultured on EphA2 (activated) or HER2 (non-activated) protein-coated plates. PBS treated plates served as controls. (**A**) After 72 hours, expression of EphA2-ENG was determined by qRT-PCR (mean + SD; n=3). (**B**) IPNγ production was determined by ELISA after 24 hours (mean + SD; n=3). (**C**) U373.eGFP.ffLuc or BV173.ffLuc cells were cocultured with NT T-cells and increasing numbers of transwell-separated activated or non-activated EphA2-ENG T-cells. After 48 hours, live tumor cells were determined by luciferase assay (n=3; duplicate assay for each donor; activated vs. non-activated T-cells: for U373: p<0.00001; for BV173: p=0.12).

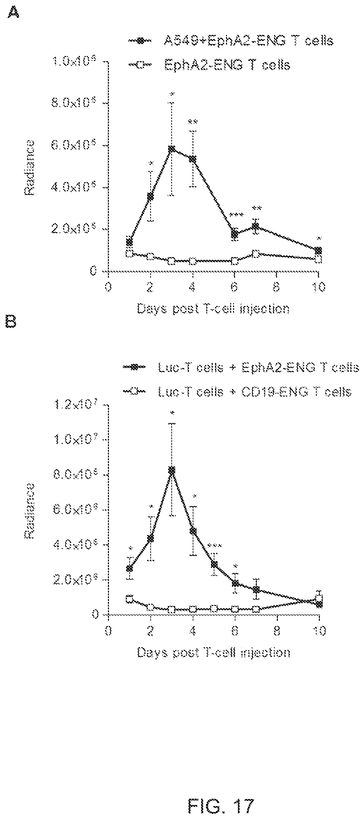

**FIG. 17** demonstrates antigen-dependent expansion of EphA2-ENG T-cells and bystander T-cells *in vivo.* (**A**) A549 tumor- or non-tumor-bearing mice received an i.v. injection of an admixture of 5x10⁶ eGFP.ffLuc-expressing EphA2-ENG and 5x10⁶ NT T-cells, and one i.p. dose of IL2 (1,500 units). Serial bioluminescence imaging was performed to track T-cells (means +/- SD are shown; n=5 per group; * p<0.05, ** p<0.01, *** p<0.005). (**B**) A549 tumor-bearing mice received an i.v. injection of an admixture of 5x10⁶ EphA2-ENG and eGFP.ffLuc-expressing 5x10⁶ NT-cells or 5x10⁶ CD19-ENG and eGFP.ffLuc-expressing 5x10⁶ NT-cells, and one i.p. dose of IL2 (1,500 units). Serial bioluminescence imaging was performed to track T-cells.

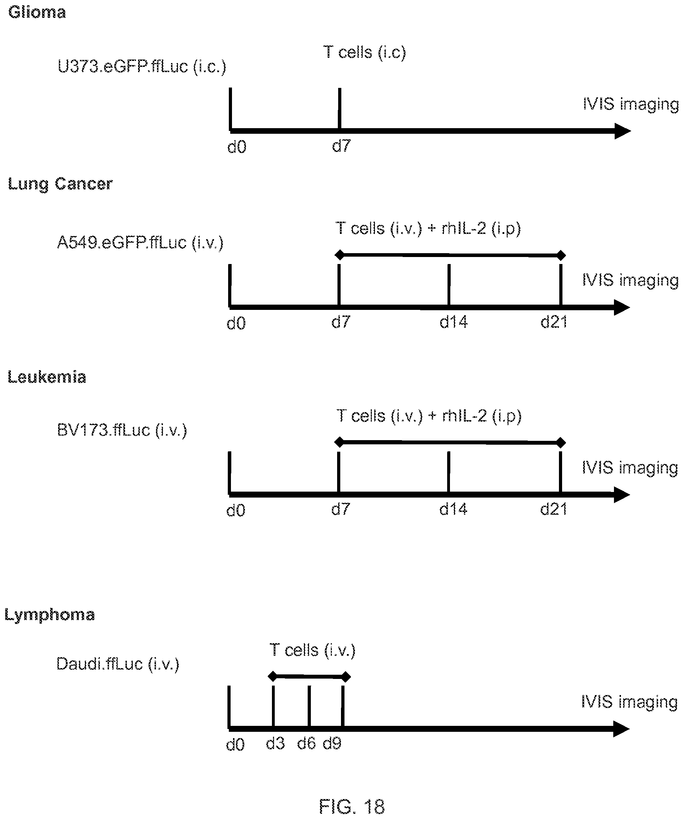

**FIG. 18** outlines the experimental schemes of the animal models in which EphA2-ENG and CD19-ENG T-cells were tested.

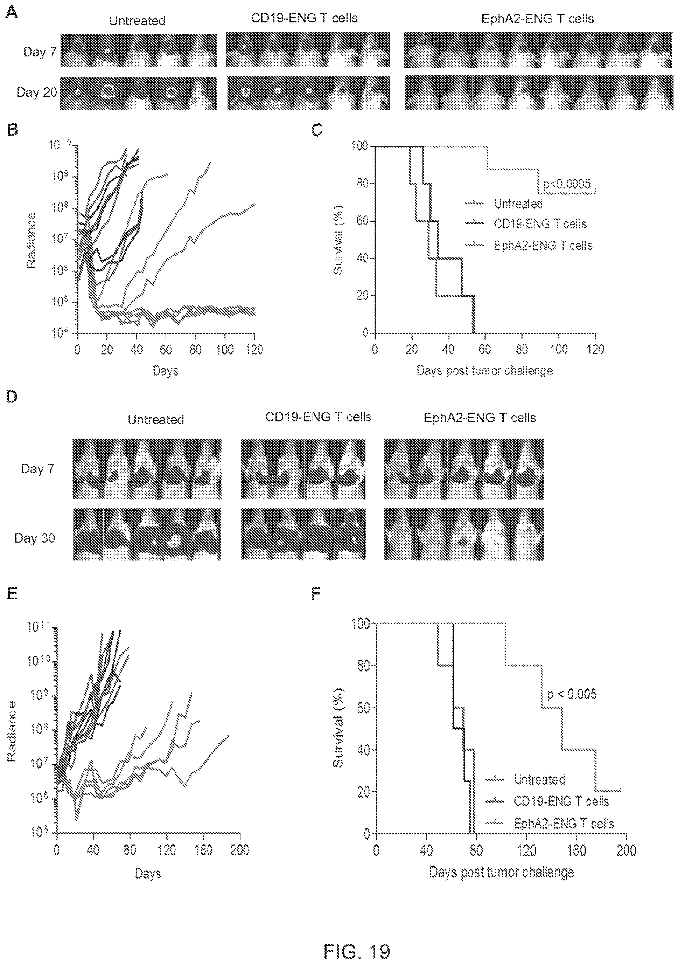

**FIG. 19** demonstrates that EphA2-ENG T-cells have potent antitumor activity *in vivo.* (**A-C**) Antitumor activity of EphA2-ENG T-cells in U373 glioma SCID xenograft model. Seven days after intracranial injection of 1x10⁵ U373.eGFP.ffLuc cells, 2x10⁶ EphA2-ENG (n=8) or CD19-ENG (n=5) T-cells were injected intracranially in the same site. Untreated animals served as controls (n=5). Tumor growth was followed by bioluminescence imaging. (**A**) Images of representative animals; (**B**) Quantitative bioluminescence imaging results for each mice (radiance=photons/sec/cm²/sr) over time; (**C**) Kaplan-Meier survival curve. (**D-F**) Antitumor activity of EphA2-ENG T-cells in A549 lung tumor SCID xenograft model. Seven, 14, and 21 days after i.v. injection of 2.5x10⁶ A549.eGFP.ffLuc cells mice received an i.v. dose of 1x10⁷ EphA2-ENG (n=5) or CD19-ENG T-cells (n=4) and an i.p. dose of IL2 (1,500 units). Untreated animals served as controls (n=5). Tumor growth was followed by bioluminescence imaging. (**D**) Images of representative animals; (**E**) Quantitative bioluminescence imaging results for each mice; (**F**) Kaplan-Meier survival curve.

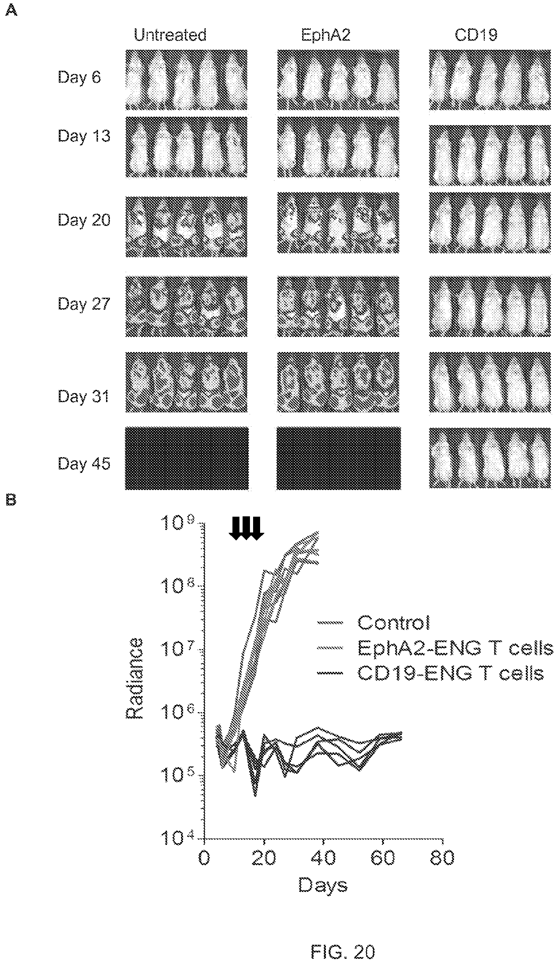

**FIG. 20** demonstrates that CD19-ENG T-cells have potent antitumor activity in a leukemia xenograft model. BV173.ffLuc cells were injected i.v. on day 0, and on day 7, 14, 21 mice received 1x10⁷ EphA2-ENG T-cells (n=5) or CD19-ENG T-cells (n=5) i.v. with one i.p. dose of IL2. Untreated animals served as controls (n=5). (**A**) Representative images, (**B**) Quantitative bioluminescence.

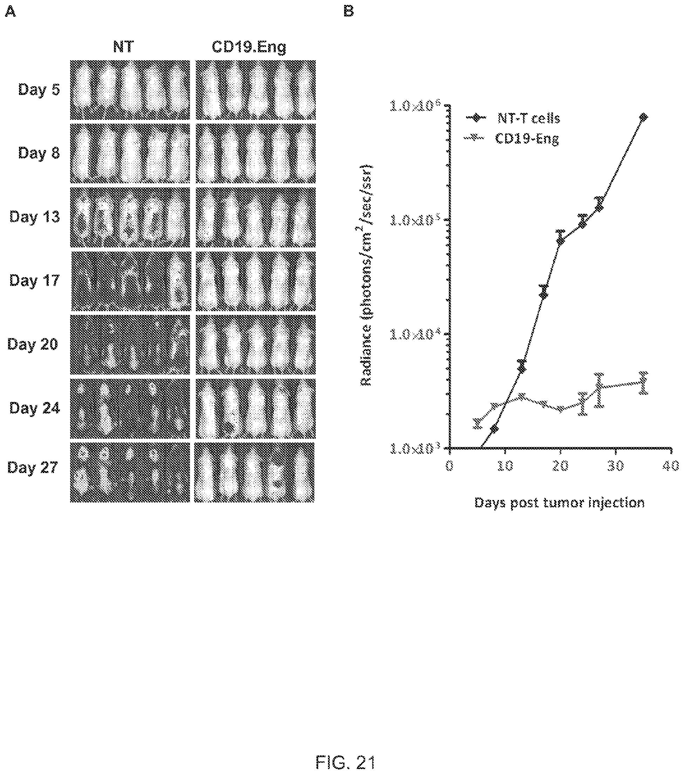

**FIG. 21** demonstrates that CD19-ENG T-cells have potent antitumor activity in a lymphoma xenograft model. Daudi.ffLuc cells were injected i.v. on day 0, and on day 3, 6, 9 mice received 1x10⁷ CD19-ENG T-cells (n=5) or non-transduced (NT) T-cells (n=5) i.v. (**A**) Representative images, (**B**) Quantitative bioluminescence.
**FIG. 22** demonstrates that T-cells that express a CD19-specific engager molecule and the co- stimulatory molecules CD80 and 4-1BBL consistently produce IL-2 in coculture assays. (**A**) Scheme of used retroviral vectors and CD19-ENG/Co-stim T-cells. (**B**) Expression of CD80 and 4-1BBL on CD19-ENG and CD19-ENG/Co-stim T-cells; n=4. (**C**) Consistent IL-2 production post stimulation with BV173 (CD80-/CD86-) of CD19-ENG/Co-stim T-cells; n=2. No IL-2 production was observed with Co-stim T-cells expressing an irrelevant engager molecule (EphA2-ENG/Co-stim T-cells), confirming antigen-specific IL-2 secretion.

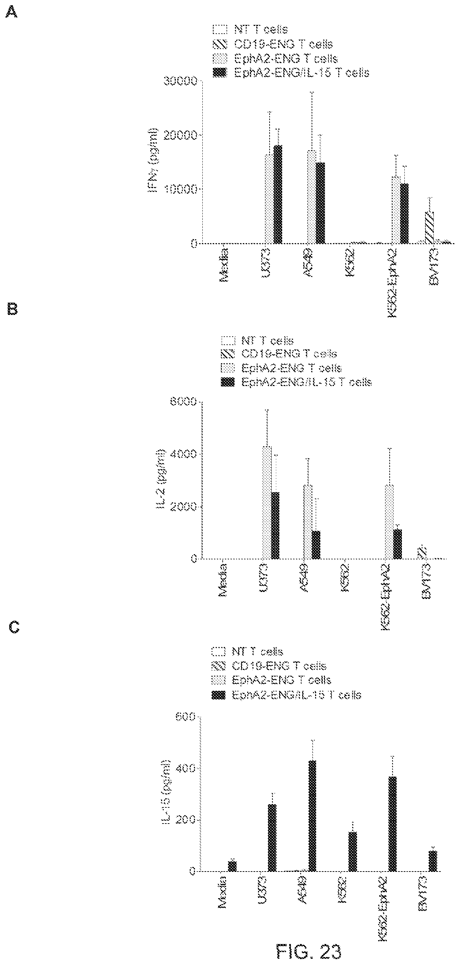

**FIG. 23** demonstrate that T cells that are genetically modified with retroviral vectors encoding an EphA2-ENG and IL15 express not only IFNg and IL2 post activation but also increased level of IL15. NT T cells, CD19-ENG T cells, EphA2-ENG T cells, or EphA2-ENG/IL15 T cells were cocultured with EphA2-positive/CD19-negative cells (U373, A549, K562-EphA2), EphA2-negative/CD19-negative cells (K562) or EphA2-negative/CD19-positive cells (BV173).. After 24 hours, IFNγ (**A**), IL2 (**B**), and IL15 (**B**) production was determined by ELISA (n=4).

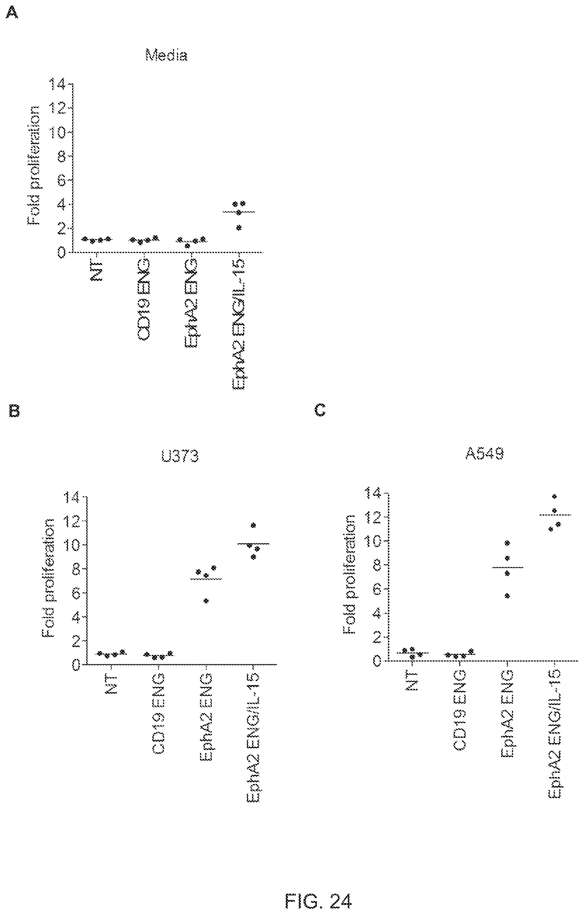

**FIG. 24** demonstrate that T cells that are genetically modified with retroviral vectors encoding an EphA2-ENG and IL15 have greater proliferative potential than T cells that are only modified with EphA2-ENG. NT T cells, CD19-ENG, EphA2-ENG, and EphA2/IL15 T-cells were cocultured with EphA2-positive (U373, A549) tumor cells (**B, C**) at a ratio of 10:1 or media (**A**). After 7 days, number of viable T-cells were enumerated by trypan blue staining (U373: EphA2-ENG vs EphA2-ENG/IL15 p=0.01; A549: EphA2-ENG vs EphA2-ENG/IL15 p=0.008; n=4).

### DETAILED DESCRIPTION OF THE INVENTION

As used herein, the words "a" and "an" when used in the present specification in concert with the word comprising, including the claims, denote "one or more." Some aspects described herein may consist of or consist essentially of one or more elements, method steps, and/or methods of the invention. It is contemplated that any method or composition described herein can be implemented with respect to any other method or composition described herein.

As used herein, the term "engager" or "engager molecule" refers to a molecule that is secreted from a cell that activates immune cells. In particular embodiments, the engager activates specific immune cells according to the domains present in the engager. Illustrative examples of cells that secrete engagers, but are not limited to, include T-cells, NK cells, NKT cells, CAR T-cells, mesenchymal stem cells (MSCs), neuronal stem cells, hematopoietic stem cells, or a mixture thereof, in some cases.

As used herein, the term "Antigen recognition domain" refers to a part of the engager molecule that recognizes an antigen. In particular aspects described herein, antigens can be of any nature including, but not limited to, proteins, carbohydrates, and/or synthetic molecules.

As used herein, the term "activation domain" refers to a part of the engager molecules that interacts with the immune cells and induces a positive or negative immunomodulatory signal. Ilustrative examples of positive immunomodulatory signals include signals that induce cell proliferation, cytokine secretion, or cytolytic activity. Illustrative examples of negative immunomodulatory signals include signals that inhibit cell proliferation, inhibit the secretion of immunosuppressive factors, or induce cell death.

As used herein, the term "native immune cell" refers to an immune cell that naturally occurs in the immune system. Illustrative examples include, but are not limited to, T-cells, NK cells, NKT cells, B cells, and dendritic cells.

As used herein, the term "engineered immune cell" refers to an immune cell that is genetically modified.

As used herein, the term "co-stimulatory domain" or "co-stimulatory signaling domain" refers to an intracellular signaling domain of a co-stimulatory molecule. In particular aspects, it refers to a domain that provides additional signals to the immune cell in conjunction with an activation domain .Co-stimulatory molecules are cell surface molecules other than antigen receptors or Fc receptors that provide a second signal required for efficient activation and function of T lymphocytes upon binding to antigen. Illustrative examples of such co-stimulatory molecules include CD27, CD28, 4-1BB (CD137), OX40 (CD134), CD30, CD40, ICOS (CD278), LFA-1, CD2, CD7, LIGHT, NKD2C, CD70, CD80, CD86, and CD83.

### I. ENGAGER MOLECULES

In particular embodiments, cells are genetically modified (including immune cells) with engager molecules comprising at least an antigen recognition domain and an activation domain and, optionally, a cytokine, costimulatory domain, and/or domain for inhibition of negative regulatory molecules of T-cell activation. The engager molecule's antigen recognition domain binds to one or more molecules present in and/or on target cells or that are secreted by target cells. In specific aspects, the target cells are cancer cells, including at least solid tumor cells. Once engager molecules have bound a target molecule, they can activate cells that express the molecule recognized by the activation domain. Engager molecules can activate cells that are genetically modified with engager molecules or can activate unmodified cells. Depending on the desired effect, activation can result in a positive or negative signal. Example of positive signals include signals that induce cell proliferation, cytokine secretion, or cytolytic activity. Examples of negative signals include signals that inhibit T-cell proliferation, inhibit the secretion of immunosuppressive factors, or induce cell death.

In particular aspects, immune cells that secrete engager molecules are able to redirect resident (naturally endogenous to a specific individual) immune cells to cancer cells.

Aspects of the disclosure provide delivery of modified immune cells that secrete an engager molecule to an individual in need thereof (known to have cancer or suspected of having cancer, including a particular cancer) in contrast to delivering the engager molecule only to the individual itself (in the absence of being produced by modified immune cells). In the present disclosure, the individual receives the modified immune cells that allow production of the engager molecule(s). In particular aspects, the cells produce immunostimulatory cytokines; proliferate in an antigen-specific manner; kill the appropriate target cells; redirect bystander immune cells (including at least T-cells or NK cells) to cancer cells; secrete engager molecules upon activation; and/or are effective against cancer in a loco-regional or systemic manner. FIG. 3 illustrates examples of modified T-cells or NK cells that secrete engager molecules. Although a particular T-cell or NK-cell may produce an engager that can target the same cancer cell-specific antigen, the activation domains for a T-cell or NK cell must be different, because NK cells do not express CD3. Examples of activation domains for a NK cell include at least CD16, NKG2D, or NKp30, for example.

In various aspects described herein, methods and compositions relate to compositions comprising a bispecific single chain antibody construct, or a bispecific constructs that has ligands, peptides, or soluble TCRs as antigen recognition domains and/or ligands as immune cell activation domains instead of fragments derived from antibodies (**FIG. 3**). The bispecific single chain antibody construct is characterized to comprise or consist of or consist essentially of at least two domains, whereby one of said at least two domains specifically binds to a tumor antigen listed in **Table 1,** for example (EphA2, CD19, CD123, LeY, B7H3, HER2, and EGFR, (including EGFRvIII), and a second domain binds to an immune cell surface protein, such as CD3 on T-cells, for example. In addition, an engager molecule may comprise three or more domains. Aspects described herein further encompass a process for the production of the composition of the disclosure, a method for the prevention, treatment or amelioration of cancer, and the use of a bispecific engager molecule construct in the prevention, treatment or amelioration of cancer or at least one symptom thereof.

In some aspects described herein, the engager compositions comprise a bispecific single chain antibody construct in addition to a third domain (although in some cases, more domains may be added), which may be referred to as a tripartite engager. The third or more domain may enhance activity of the composition or method, such as by providing a cytokine, costimulatory domain, and/or domain for inhibition of negative regulatory molecules of T-cell activation. Aspects described herein further encompass a process for the production of the tripartite engager, a method for the prevention, treatment or amelioration of cancer, and the use of the tripartite engager in the prevention, treatment or amelioration of cancer.

In particular embodiments, an engager molecule comprises an antigen recognition domain that binds EphA2. EphA2 may be referred to as EPH receptor A2 (ephrin type-A receptor 2; EPHA2; ARCC2; CTPA; CTPP1; or ECK), which is a protein that in humans is encoded by the EPHA2 gene in the ephrin receptor subfamily of the protein-tyrosine kinase family. Receptors in this subfamily generally comprise a single kinase domain and an extracellular region comprising a Cys-rich domain and 2 fibronectin type III repeats; embodiments of the antibodies of the disclosure may target any of these domains. The ephrin receptors are divided into two groups as a result of the similarity of their respective extracellular domain sequences and also their affinities for binding ephrin-A and ephrin-B ligands, and EphA2 encodes a protein that binds ephrin-A ligands. An exemplary human EphA2 nucleic sequence is in GenBank® Accession No. NM_004431, and an exemplary human EphA2 polypeptide sequence is in GenBank® Accession No. NP_004422.

In particular embodiments, an engager molecule comprises an antigen recognition domain that binds CD19. CD19 may be referred to as CD19 molecule, CD19 antigen, Differentiation antigen CD19, B-lymphocyte surface antigen B4, CVID3. Information in regards to gene locus, nucleotide and amino acid sequences can be found at HGNC: 1633, Entrez Gene: 930, Ensembl: ENSG00000177455, UniProtKB: P15391. CD19 encodes a cell surface molecule, which assembles with the antigen receptor of B lymphocytes in order to decrease the threshold for antigen receptor-dependent stimulation. An exemplary human CD19 molecule is in GenBank® Accession No. NM_001178098, and an exemplary human CD19 polypeptide is in GenBank® Accession No. NP_001171569.

The engager cells may be generated by any suitable method in the art. In specific embodiments, the engager T-cells are generated by viral transduction of T-cells, although viral transduction of NK cells and other cells (CAR T-cells, NKT-cells, MSCs, neuronal stem cells, hematopoietic stem cells, in some cases) in at least certain cases is also useful, in some aspects described herein. The endogenous, native TCR of the transduced T-cells can either be unspecific or specific for an antigen, such as a tumor antigen or a viral antigen.

### A. Antigen Recognition Domain

The engager compositions of the disclosure include an antigen recognition domain that allows the engager-expressing T-cell and the corresponding engager molecule to target a particular cell of interest that expresses the antigen or to target a secretable antigen. In particular aspects, any cell may comprise the antigen, but in specific aspects the antigen is displayed on a cancer cell, including a solid tumor cell. The cancer cell antigen may be of any kind, but in particular aspects it is specific to a cancer cell and may be specific to a particular type of cancer cell. For example, in embodiments wherein EphA2 is utilized, the cancer cell may be breast, lung, prostate, or glioblastoma. The cell may be also be a pathogenic cell, such as a bacterial cell.

Any cancer antigen may be targeted by the engager-expressing T-cells described herein or the corresponding engager molecules thereof. In specific aspects, the antigens are listed in Table 1. In particular aspects, the engager molecule comprises more than one antigen recognition domain.

When targeting a particular cancer antigen, the antigen may be targeted with any suitable scFv or antigen binding fragment of an antibody. Examples of particular scFvs, which were used to construct engager molecules are listed in **Table 2.** The functionality of T-cells expressing the respective engager molecules was confirmed, and is described in detail in EXAMPLE 2.

**Table 2: scFVs used for engager molecule construction**

| Target | MAb |
|---|---|
| CD19 | FMC63 |
| CD123 | 26292 |
| CD123 | 32716 |
| LeY | Hu3S193 |
| EphA2 | 4H5 |
| B7H3 | 8H9 |
| HER2 | FRP5 |
| EGFR including EGFRvIII | 806 |

Other potential antigens are listed in **Table 1** and/or are discussed elsewhere herein.

### B. Activation Domain

The engager compositions of the disclosure include an activation domain that allows the immune cell that expresses the engager molecule and/or other immune cells to bind to the engager and a target cell. The activation domain is an antibody or antigen-binding fragment thereof, in particular aspects. Illustrative examples of activation domains include, but are not limited to antibody or antigen-binding fragment thereof, ligands, peptides, soluble T-cell receptors, or combinations thereof.

The immune cell to which the engager binds may be an unmodified naturally endogenous (to the recipient individual) immune cell, or it may be a genetically modified immune cell. Binding of the engager to the target immune cell through the activation domain (such as an CD3 monoclonal antibody in the case of T-cells), thereby activates the target immune cell. Other activation molecules that can be readily targeted with engagers include co-stimulatory molecules such as CD27, CD28, CD134, and CD137. For example, T-cells can be engineered to express one engager molecule with an EphA2-specific antigen recognition domain and a CD3-specific activation domain, and another engager with a HER2-specific antigen recognition domain and a CD28-specific activation domain. These engager T-cells would only be fully activated at tumor sites at which both antigens are expressed. When the engager is to target NK cells, the activation domain may comprise of an antibody that recognizes CD 16 (such as NM3E2 antibody), or ligands specific for NKG2D (ULBP2), or NKp30 (B7H6). In specific embodiments, the activation domain comprises ligands, receptors, peptides, *etc.*

### C. Optional Additional Domains

In particular embodiments, an engager comprises an additional domain that enhances the activity of the engager and/or enhances the activity of the immune cell expressing the engager molecule. Although an additional domain may be of any kind, in specific embodiments the additional domain comprises one or more antigen recognition domains or one or more activation domain. The additional domain may comprise a cytokine, a co-stimulatory domain, or an entity that inhibits negative regulatory molecules of T-cell activation. In some embodiments, only one additional domain is employed in engager molecules, but in other embodiments more than one may be employed, including more than one of the same or different type.

Additional domains could offset immune escape by targeting an additional antigen. For example, an engager molecule could be designed to target CD19 and CD22 for hematological malignancies or EphA2 and HER2 for solid tumors. Additional domains could also provide co-stimulation. For example, an engager molecule could be designed to target a tumor antigen (see Table 1), and contain a CD3-specific scFv for T-cell activation and the extracellular domain of 41BBL to provide co-stimulation. An additional domain could attract immune cells. For example, an additional domain encoding the chemokine RANTES could be used to increase the trafficking of immune cells to tumor sites. An additional domain could also be used to provide an immune cell growth factor such a cytokine. For example, an additional domain encoding cytokines like IL-2 or IL15 could be used to enhance immune cell proliferation and expansion. An additional domain could also be used to change the physical properties of the engager molecule. For example, an additional domain encoding a CH2-CH3 domain or a leucine zipper could promote dimerization or trimerization of the engager molecule resulting in enhanced function.

In particular embodiments, the additional domain encodes any cytokine. In specific aspects, the cytokine may be IL-2, IL-7, and/or IL-15, and in some aspects, an engager comprises more than one additional domain encoding cytokines.

In particular embodiments, an engager comprises a co-stimulatory domain as described elsewhere herein. In particular embodiments, however, the co-stimulatory domain comprises CD80, CD137, and the like.

In particular aspects, the engager molecule comprises a domain that inhibits negative regulatory molecules of T-cell activation. In specific aspects, the domain is PD-L1 or CTLA4, for example.

### D. Soluble T-cell Receptor Domain

In some aspects described herein, instead of an antigen recognition domain being an antibody, an engager comprises a different kind of domain that is not an antibody but that is capable of recognizing a cancer cell. In one aspect, the engager comprises one member of a ligand-receptor binding pair, wherein the cognate member is expressed on the cancer cell. In certain aspects, the engager comprises a soluble T-cell receptor (TCR) domain, such as in lieu of an antibody. **FIG. 2** illustrates an exemplary embodiment with a T-cell activation domain linked to a soluble TCR.

### E. Examples of Specific Engager Molecules

Below are provided specific examples of engager molecules. In general, an scFv contains a VH and VL domain connected by a linker peptide. For example, SEQ ID NO: 1 is a CD19-CD3 T-cell Engager comprising the following formula:

### 1. CD19-CD3 T-cell Engager

Leader-VL FMC63-(G4S1)3-VH FMC63-SG4S-VHOKT3-(G4S1)3-VLOKT3

SEQ ID NO:1 is as follows, where the first underlined section is the leader and the following underlined sections are linker sequences between the respective scFvs, according to the formula above:

MDWIWRILFLVGAATGAHSDIQMTQTTSSLSASLGDRVTISCRASQDISKYLNWYQQK
PDGTVKLLIYHTSRLHSGVPSRFSGSGSGTDYSLTISNLEQEDIATYFCQQGNTLPYTFGG
GTKLELKRGGGGSGGGGSGGGGSGGGGSEVQLQQSGPGLVAPSQSLSVTCTVSGVSL
PDYGVSWIRQPPRKGLEWLGVIWGSETTYYNSALKSRLTIIKDNSKSQVFLKMNSLQTD
DTAIYYCAKHYYYGGSYAMDYWGQGTTVTVSSYVTVSSSGGGGSDIKLQQSGAELAR
PGASVKMSCKTSGYTFTRYTMHWVKQRPGQGLEWIGYINPSRGYTNYNQKFKDKATL
TTDKSSSTAYMQLSSLTSEDSAVYYCARYYDDHYCLDYWGQGTTLTVSSGGGGSGGG
GSGGGGSDIQLTQSPAIMSASPGEKVTMTCRASSSVSYMNWYQQKSGTSPKRWIYDTS
KVASGVPYRFSGSGSGTSYSLTISSMEAEDAATYYCQQWSSNPLTFGAGTKLELKS

### 2. IL3Ra-CD3 T-cell Engager

Leader-VH26292-(G4S1)3-VL26292-SG4S-VHOKT3-(G4S1)3-VLOKT3

SEQ ID NO:2 is as follows, where the first underlined section is the leader and the following underlined sections are linker sequences between the respective scFvs according to the formula above:

MDWIWRILFLVGAATGAHSQVQLQQPGAELVRPGASVKLSCKASGYTFTSYWMNWV
KQRPDQGLEWIGRIDPYDSETHYNQKFKDKAILTVDKSSSTAYMQLSSLTSEDSAVYYC
ARGNWDDYWGQGTTLTVSSGGGGSGGGGSGGGGSDVQITQSPSYLAASPGETITINC
RASKSISKDLAWYQEKPGKTNKLLIYSGSTLQSGIPSRFSGSGSGTDFTLTISSLEPEDFAM
YYCQQHNKYPYTFGGGTKLEIKSGGGGSDIKLQQSGAELARPGASVKMSCKTSGYTFT
RYTMHWVKQRPGQGLEWIGYINPSRGYTNYNQKFKDKATLTTDKSSSTAYMQLSSLTS
EDSAVYYCARYYDDHYCLDYWGQGTTLTVSSGGGGSGGGGSGGGGSDIQLTQSPAI
MSASPGEKVTMTCRASSSVSYMNWYQQKSGTSPKRWIYDTSKVASGVPYRFSGSGSGT
SYSLTISSMEAEDAATYYCQQWSSNPLTFGAGTKLELKS

### 3. IL3Ra-CD3 T-cell Engager

Leader-VH32716-(G4S1)3-VL32716-SG4S-VHOKT3-(G4S1)3-VLOKT3

SEQ ID NO:3 is as follows, where the first underlined section is the leader and the following underlined sections are linker sequences between the respective scFvs according to the formula above:

MDWIWRILFLVGAATGAHSQIQLVQSGPELKKPGETVKISCKASGYIFTNYGMNWVK
QAPGKSFKWMGWINTYTGESTYSADFKGRFAFSLETSASTAYLHINDLKNEDTATYFCA
RSGGYDPMDYWGQGTSVTVSSGGGGSGGGGSGGGGSDIVLTQSPASLAVSLGQRATI
SCRASESVDNYGNTFMHWYQQKPGQPPKLLIYRASNLESGIPARFSGSGSRTDFTLTINP
VEADDVATYYCQQSNEDPPTFGAGTKLELKSGGGGSDIKLQQSGAELARPGASVKMSC
KTSGYTFTRYTMHWVKQRPGQGLEWIGYINPSRGYTNYNQKFKDKATLTTDKSSSTAY
MQLSSLTSEDSAVYYCARYYDDHYCLDYWGQGTTLTVSSGGGGSGGGGSGGGGSDI
QLTQSPAIMSASPGEKVTMTCRASSSVSYMNWYQQKSGTSPKRWIYDTSKVASGVPYR
FSGSGSGTSYSLTISSMEAEDAATYYCQQWSSNPLTFGAGTKLELKS

### 4. Le Y-CD3 T-cell Engager

Leader-VHHu3S193-(G4S1)3-VLHu3S193-SG4S-VHOKT3-(G4S1)3-VLOKT3

SEQ ID NO:4 is as follows, where the first underlined section is the leader and the following underlined sections are linker sequences between the respective scFvs according to the formula above:

MDWIWRILFLVGAATGAHSEVQLVESGGGVVQPGRSLRLSCSTSGFTFSDYYMYWVR
QAPGKGLEWVAYMSNVGAITDYPDTVKGRFTISRDNSKNTLFLQMDSLRPEDTGVYFC
ARGTRDGSWFAYWGQGTPVTVSSGGGGSGGGGSGGGGSDIQMTQSPSSLSASVGDR
VTITCRSSQRIVHSNGNTYLEWYQQTPGKAPKLLIYKVSNRFSGVPSRFSGSGSGTDFTFT
ISSLQPEDIATYYCFQGSHVPFTFGQGTKLQITSGGGGSDIKLQQSGAELARPGASVKMS
CKTSGYTFTRYTMHWVKQRPGQGLEWIGYINPSRGYTNYNQKFKDKATLTTDKSSSTA
YMQLSSLTSEDSAVYYCARYYDDHYCLDYWGQGTTLTVSSGGGGSGGGGSGGGGSD
IQLTQSPAIMSASPGEKVTMTCRASSSVSYMNWYQQKSGTSPKRWIYDTSKVASGVPYR
FSGSGSGTSYSLTISSMEAEDAATYYCQQWSSNPLTFGAGTKLELKS

### 5. EphA2-CD3 T-cell Engager

Leader-VH4H5-(G4S1)3-VL4H5-SG4S-VHOKT3-(G4S1)3-VLOKT3

SEQ ID NO:5 is as follows, where the first underlined section is the leader and the following underlined sections are linker sequences between the respective scFvs according to the formula above:

MDWIWRILFLVGAATGAHSQVQLLESGGGLVQPGGSLRLSCAASGFTFSSYTMSWVR
QAPGQALEWMGTISSGGTYTYYPDSVKGRFTISRDNAKNSLYLQMNSLRAEDTAVYYC
AREAIFTYWGRGTLVTSSGGGGSGGGGSGGGGSDIQLTQSPSSLSASVGDRVTITCKAS
QDINNYLSWYQQKPGQAPRLLIYRANRLVDGVPDRFSGSGYGTDFTLTINNIESEDAAY
YFCLKYDVFPYTFGQGTKVEIKSGGGGSDIKLQQSGAELARPGASVKMSCKTSGYTFTR
YTMHWVKQRPGQGLEWIGYINPSRGYTNYNQKFKDKATLTTDKSSSTAYMQLSSLTSE
DSAVYYCARYYDDHYCLDYWGQGTTLTVSSGGGGSGGGGSGGGGSDIQLTQSPAIM
SASPGEKVTMTCRASSSVSYMNWYQQKSGTSPKRWIYDTSKVASGVPYRFSGSGSGTS
YSLTISSMEAEDAATYYCQQWSSNPLTFGAGTKLELKS

### 6. B7H3-CD3 T-cell Engager

Leader-VH8H9-(G4S1)3-VL8H9-SG4S-VHOKT3-(G4S1)3-VLOKT3

SEQ ID NO:6 is as follows, where the first underlined section is the leader and the following underlined sections are linker sequences between the respective scFvs according to the formula above:

MDWIWRILFLVGAATGAHSQVKLQQSGAELVKPGASVKLSCKASGYTFTNYDINWV
RQRPEQGLEWIGWIFPGDGSTQYNEKFKGKATLTTDTSSSTAYMQLSRLTSEDSAVYFC
ARQTTATWFAYWGQGTTVTVSSDGGGSGGGGSGGGGSDIELTQSPTTLSVTPGDRVS
LSCRASQSISDYLHWYQQKSHESPRLLIKYASQSISGIPSRFSGSGSGSDFTLSINSVEPED
VGVYYCQNGHSFPLTFGAGTKLELKQAASGGGGSDIKLQQSGAELARPGASVKMSCKT
SGYTFTRYTMHWVKQRPGQGLEWIGYINPSRGYTNYNQKFKDKATLTTDKSSSTAYM
QLSSLTSEDSAVYYCARYYDDHYCLDYWGQGTTLTVSSGGGGSGGGGSGGGGSDIQL
TQSPAIMSASPGEKVTMTCRASSSVSYMNWYQQKSGTSPKRWIYDTSKVASGVPYRFS
GSGSGTSYSLTISSMEAEDAATYYCQQWSSNPLTFGAGTKLELKS

### 7. HER2-CD3 T-cell Engager

Leader-VHFRP5-(G4S1)3-VLFRP5-G4S-VHOKT3-(G4S1)3-VLOKT3

SEQ ID NO:7 is as follows, where the first underlined section is the leader and the following underlined sections are linker sequences between the respective scFvs according to the formula above:

MDWIWRILFLVGAATGAHSEVQLQQSGPELKKPGETVKISCKASGYPFTNYGMNWV
KQAPGQGLKWMGWINTSTGESTFADDFKGRFDFSLETSANTAYLQINNLKSEDMATYF
CARWEVYHGYVPYWGQGTTVTVSSGGGGSGGGGSGGGGSDIQLTQSHKFLSTSVGD
RVSITCKASQDVYNAVAWYQQKPGQSPKLLIYSASSRYTGVPSRFTGSGSGPDFTFTISS
VQAEDLAVYFCQQHFRTPFTFGSGTKLEIKALGGGGSDIKLQQSGAELARPGASVKMSC
KTSGYTFTRYTMHWVKQRPGQGLEWIGYINPSRGYTNYNQKFKDKATLTTDKSSSTAY
MQLSSLTSEDSAVYYCARYYDDHYCLDYWGQGTTLTVSSGGGGSGGGGSGGGGSDI
QLTQSPAIMSASPGEKVTMTCRASSSVSYMNWYQQKSGTSPKRWIYDTSKVASGVPYR
FSGSGSGTSYSLTISSMEAEDAATYYCQQWSSNPLTFGAGTKLELKS

### 8. EGFR-CD3 T-cell Engager

Leader-VH806-(G4S1)3-VL806-SG4S-VHOKT3-(G4S1)3-VLOKT3

SEQ ID NO:8 is as follows, where the first underlined section is the leader and the following underlined sections are linker sequences between the respective scFvs according to the formula above:

MDWIWRILFLVGAATGAHSDVQLQESGPSLVKPSQSLSLTCTVTGYSITSDFAWNWIR
QFPGNKLEWMGYISYSGNTRYNPSLKSRISITRDTSKNQFFLQLNSVTIEDTATYYCVTA
GRGFPYWGQGTLVTVSAGGGGSGGGGSGGGGSDILMTQSPSSMSVSLGDTVSITCHSS
QDINSNIGWLQQRPGKSFKGLIYHGTNLDDEVPSRFSGSGSGADYSLTISSLESEDFADY
YCVQYAQFPWTFGGGTKLEIKRSGGGGSDIKLQQSGAELARPGASVKMSCKTSGYTFT
RYTMHWVKQRPGQGLEWIGYINPSRGYTNYNQKFKDKATLTTDKSSSTAYMQLSSLTS
EDSAVYYCARYYDDHYCLDYWGQGTTLTVSSGGGGSGGGGSGGGGSDIQLTQSPAI
MSASPGEKVTMTCRASSSVSYMNWYQQKSGTSPKRWIYDTSKVASGVPYRFSGSGSGT
SYSLTISSMEAEDAATYYCQQWSSNPLTFGAGTKLELKS

### 9. CD16-EphA2 NK-cell Engager

Leader-VHNM3E2-(G4S1)3-VLNM3E2-SG4S-VH4H5-(G4S1)3-VL4H5

SEQ ID NO:9 is as follows, where the first underlined section is the leader and the following underlined sections are linker sequences between the respective scFvs according to the formula above:

MDWIWRILFLVGAATGAHSEVQLVESGGGVVRPGGSLRLSCAASGFTFDDYGMSWV
RQAPGKGLEWVSGINWNGGSTGYADSVKGRFTISRDNAKNSLYLQMNSLRAEDTAVY
YCARGRSLLFDYWGQGTLVTVSRGGGGSGGGGSGGGGSGGGGSSSELTQDPAVSVA
LGQTVRITCQGDSLRSYYASWYQQKPGQAPVLVIYGKNNRPSGIPDRFSGSSSGNTASLT
ITGAQAEDEADYYCNSRDSSGNHVVFGGGTKLTVGSGGGGSQVQLLESGGGLVQPGG
SLRLSCAASGFTFSSYTMSWVRQAPGQALEWMGTISSGGTYTYYPDSVKGRFTISRDNA
KNSLYLQMNSLRAEDTAVYYCAREAIFTYWGRGTLVTSSGGGGSGGGGSGGGGSDIQ
LTQSPSSLSASVGDRVTITCKASQDINNYLSWYQQKPGQAPRLLIYRANRLVDGVPDRFS
GSGYGTDFTLTINNIESEDAAYYFCLKYDVFPYTFGQGTKVEIK

### F. Engagers - General Concepts

The term "bispecific single chain antibody construct" relates to a construct comprising two antibody derived binding domains. One of the binding domains may comprise variable regions (or parts thereof) of an antibody, antibody fragment or derivative thereof, capable of specifically binding to/interacting with a target antigen, *e*.*g*.,EphA2 or CD19. The second binding domain may comprise variable regions (or parts thereof) of an antibody, antibody fragment or derivative thereof, capable of specifically binding to/interacting with an activation molecule, e.g., human CD3 antigen. In specific embodiments, a part of a variable region comprises at least one CDR ("Complementary determining region"), such as at least a CDR1, CDR2, or CDR3 region. The two domains/regions in the single chain antibody construct are preferably covalently connected to one another as a single chain.

An scFv in general contains a VH and VL domain connected by a linker peptide. The secretable engager is composed of a signal peptide (to allow for secretion) from cells, followed by two or more scFvs connected by linker peptides (Lx, Ly, Lz). Linkers may be of a length and sequence sufficient to ensure that each of the first and second domains can, independently from one another, retain their differential binding specificities. Bispecific scFvs can be arranged in different formats: V_{H}α-Lx-V_{L}α-Ly-V_{H}β-Lz-V_{L}β, V_{L}α-Lx-V_{H}α-Ly-V_{H}β-Lz-V_{L}β, V_{L}α-Lx-V_{H}α-Ly-V_{L}β-Lz-V_{H}β, V_{H}α-Lx-V_{L}α-Ly-V_{L}β-Lz-V_{H}β, V_{H}α-Lx-V_{L}β-Ly-V_{H}β-Lz-V_{L}α, V_{H}β-Lx-V_{L}α-Ly-V_{H}α-Lz-V_{L}β, V_{L}α-Lx-V_{H}β-Ly-V_{L}β-Lz-V_{H}α, V_{L}β-Lx-V_{H}α-Ly-V_{L}α-Lz-Y_{H}β.

In specific embodiments, the "bispecific single chain antibody construct" to be employed in the composition of the disclosure comprises a bispecific single chain Fv (scFv). Illustrative examples of bispecific single chain molecules are known in the art and are described in WO 99/54440; Mack, J. Immunol. (1997), 158, 3965-3970; Mack, PNAS, (1995), 92, 7021-7025; Kufer, Cancer Immunol. Immunother., (1997), 45, 193-197; Loffler, Blood, (2000), 95, 6, 2098-2103; and Bruhl, J. Immunol., (2001), 166, 2420-2426.

In specific aspects described herein, an exemplary molecular format of the disclosure provides a polypeptide construct wherein the antibody-derived region comprises one V_{H} and one V_{L} region. In particular aspects, the intramolecular orientation of the V_{H}-domain and the V_{L}-domain, which are linked to each other by a linker-domain, in the scFv format is not decisive for the recited bispecific single chain constructs. scFvs with both possible arrangements (V_{H}-domain-linker domain-V_{L}-domain; V_{L}-domain-linker domain-V_{H}-domain) are contemplated in particular aspects of the bispecific single chain construct.

In specific embodiments, the engager construct may also comprise additional domains, *e.g.*, the antigen binding domain may contain multiple antigen recognition binding domains allowing targeting of multiple antigens; the activation domain may contain multiple domains to activate cells.

The term "single-chain" as used in accordance with the present disclosure in some embodiments means that first and second domains of the bispecific single chain construct are covalently linked, preferably in the form of a co-linear amino acid sequence encodable by a single nucleic acid molecule.

The term "binding to/interacting with" as used in the context with the present disclosure defines a binding/interaction of at least two "antigen-interaction-sites" with each other. The term "antigen-interaction-site" defines, in accordance with the present disclosure, a motif of a polypeptide that shows the capacity of specific interaction with a specific antigen or a specific group of antigens. The binding/interaction is also understood to define a "specific recognition". The term "specifically recognizing" means in accordance with this disclosure that the antibody molecule is capable of specifically interacting with and/or binding to at least two amino acids of each of the target molecules as defined herein. The term relates to the specificity of the antibody molecule, *i.e.* to its ability to discriminate between the specific regions of the human target molecule as defined herein. The specific interaction of the antigen-interaction-site with its specific antigen may result in an initiation of a signal, e.g. due to the induction of a change of the conformation of the antigen, an oligomerization of the antigen, *etc.* Further, the binding may be exemplified by the specificity of a "key-lock-principle". Thus, specific motifs in the amino acid sequence of the antigen-interaction-site and the antigen bind to each other as a result of their primary, secondary or tertiary structure as well as the result of secondary modifications of said structure, in some embodiments. The specific interaction of the antigen-interaction-site with its specific antigen may result as well in a simple binding of the site to the antigen.

The term "specific interaction" as used in accordance with the present disclosure means that the bispecific single chain construct does not or essentially does not cross-react with (poly)peptides of similar structures. Cross-reactivity of a panel of bispecific single chain constructs under investigation may be tested, for example, by assessing binding of the panel of bispecific single chain construct under conventional conditions (see, *e.g*., Harlow and Lane, Antibodies: A Laboratory Manual, Cold Spring Harbor Laboratory Press, 1988 and Using Antibodies: A Laboratory Manual, Cold Spring Harbor Laboratory Press, 1999) to the (poly)peptide of interest as well as to a number of more or less (structurally and/or functionally) closely related (poly)peptides. Only those antibodies that bind to the (poly)peptide/protein of interest but do not or do not essentially bind to any of the other (poly)peptides are considered specific for the (poly)peptide/protein of interest. Examples for the specific interaction of an antigen-interaction-site with a specific antigen comprise the specificity of a ligand for its receptor. The definition particularly comprises the interaction of ligands which induce a signal upon binding to its specific receptor. Examples for corresponding ligands comprise cytokines that interact/bind with/to its specific cytokine-receptors. Another example for said interaction, which is also particularly comprised by said definition, is the interaction of an antigenic determinant (epitope) with the antigenic binding site of an antibody.

The term "binding to/interacting with" may also relate to a conformational epitope, a structural epitope or a discontinuous epitope consisting of two regions of the human target molecules or parts thereof. In context of this disclosure, a conformational epitope is defined by two or more discrete amino acid sequences separated in the primary sequence which come together on the surface of the molecule when the polypeptide folds to the native protein (Sela, (1969) Science 166, 1365 and Layer, (1990) Cell 61, 553-6).

In particular embodiments, the constructs of the present disclosure are also envisaged to specifically bind to/interact with a conformational/structural epitope(s) composed of and/or comprising the two regions of the human CD3 complex described herein or parts thereof as disclosed herein below.

Accordingly, specificity can be determined experimentally by methods known in the art and methods as disclosed and described herein. Such methods comprise, but are not limited to Western blots, ELISA-, RIA-, ECL-, IRMA-, EIA-tests and peptide scans.

The term "antibody fragment or derivative thereof' relates to single chain antibodies, or fragments thereof, synthetic antibodies, antibody fragments, such as a Camel Ig, Ig NAR, Fab fragments, Fab' fragments, F(ab)'2 fragments, F(ab)'3 fragments, Fv, single chain Fv antibody ("scFv"), bis-scFv, (scFv)2, minibody, diabody, triabody, tetrabody, disulfide stabilized Fv protein ("dsFv"), and single-domain antibody (sdAb, nanobody), *etc.,* or a chemically modified derivative of any of these. Antibodies to be employed in accordance with the disclosure or their corresponding immunoglobulin chain(s) can be further modified using conventional techniques known in the art, for example, by using amino acid deletion(s), insertion(s), substitution(s), addition(s), and/or recombination(s) and/or any other modification(s) (*e.g*. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, *e.g.,* Sambrook *et al.* (1989).

The term "antibody fragment or derivative thereof' particularly relates to (poly)peptide constructs comprising at least one CDR.

Fragments or derivatives of the recited antibody molecules define (poly)peptides which are parts of the above antibody molecules and/or which are modified by chemical/biochemical or molecular biological methods. Corresponding methods are known in the art and described *inter alia* in laboratory manuals (see Sambrook et al.; Molecular Cloning: A Laboratory Manual; Cold Spring Harbor Laboratory Press, 2nd edition 1989 and 3rd edition 2001; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002).

Variable domains comprised in the herein described bispecific single chain constructs may be connected by additional linker sequences. The term "peptide linker" defines in accordance with the present disclosure an amino acid sequence by which the amino acid sequences of the first domain and the second domain of the defined construct are linked with each other. An essential technical feature of such peptide linker is that said peptide linker does not comprise any polymerization activity. The characteristics of a peptide linker, which comprise the absence of the promotion of secondary structures, are known in the art and described, *e.g.,* in Dall'Acqua et al. (Biochem. (1998) 37, 9266-9273), Cheadle et al. (Mol Immunol (1992) 29, 21-30) and Raag and Whitlow (FASEB (1995) 9(1), 73-80). An envisaged peptide linker with less than 5 amino acids can comprise 4, 3, 2 or one amino acids. A particularly preferred "single" amino acid in context of the "peptide linker" is Gly. Accordingly, the peptide linker may consist of one or more Gly residues. Furthermore, peptide linkers that also do not promote any secondary structures are preferred. The linkage of the domains to each other can be provided by, *e.g.,* genetic engineering. Methods for preparing fused and operatively linked bispecific single chain constructs and expressing them in mammalian cells or bacteria are well-known in the art (*e.g.* WO 99/54440, Ausubel, Current Protocols in Molecular Biology, Green Publishing Associates and Wiley Interscience, N.Y. 1989 and 1994 or Sambrook et al., Molecular Cloning: A Laboratory Manual, Cold Spring Harbor Laboratory Press, Cold Spring Harbor, New York, 2001).

The bispecific single chain antibody constructs described herein above and below may be humanized or deimmunized antibody constructs. Methods for the humanization and/or deimmunization of (poly)peptides and, in particular, antibody constructs are known to the person skilled in the art.

In one aspect of the pharmaceutical composition of this disclosure, the V_{H} and V_{L} regions of a human CD3 specific domain are derived from a CD3 specific antibody selected from the group consisting of X35-3, VIT3, BMA030 (BW264/56), CLB-T3/3, CRIS7, YTH12.5, F111-409, CLB-T3.4.2, TR-66, WT32, SPv-T3b, 11D8, XIII-141, XIII-46, XIII-87, 12F6, T3/RW2-8C8, T3/RW2-4B6, OKT3D, M-T301, SMC2, WT31 and F101.01. These CD3-specific antibodies are well known in the art and, inter alia, described in Tunnacliffe (1989), Int. Immunol. 1, 546-550. In specific embodiments, V_{H} and V_{L} regions are derived from antibodies/antibody derivatives and the like that are capable of specifically recognizing the human CD3-8 chain or human CD3-ζ chain.

### II. POLYNUCLEOTIDES ENCODING ENGAGERS

The present disclosure also encompasses a composition comprising a nucleic acid sequence encoding a bispecific single chain antibody construct as defined above and cells harboring the nucleic acid sequence. The nucleic acid molecule is a recombinant nucleic acid molecule, in particular aspects and may be synthetic. It may comprise DNA, RNA as well as PNA (peptide nucleic acid) and it may be a hybrid thereof.

It is evident to the person skilled in the art that one or more regulatory sequences may be added to the nucleic acid molecule comprised in the composition of the disclosure. For example, promoters, transcriptional enhancers and/or sequences that allow for induced expression of the polynucleotide of the disclosure may be employed. A suitable inducible system is for example tetracycline-regulated gene expression as described, *e.g.,* by Gossen and Bujard (Proc. Natl. Acad. Sci. USA 89 (1992), 5547-5551) and Gossen et al. (Trends Biotech. 12 (1994), 58-62), or a dexamethasone-inducible gene expression system as described, *e.g.* by Crook (1989) EMBO J. 8, 513-519.

Furthermore, it is envisaged for further purposes that nucleic acid molecules may contain, for example, thioester bonds and/or nucleotide analogues. The modifications may be useful for the stabilization of the nucleic acid molecule against endo- and/or exonucleases in the cell. The nucleic acid molecules may be transcribed by an appropriate vector comprising a chimeric gene that allows for the transcription of said nucleic acid molecule in the cell. In this respect, it is also to be understood that such polynucleotides can be used for "gene targeting" or "gene therapeutic" approaches. In another embodiment the nucleic acid molecules are labeled. Methods for the detection of nucleic acids are well known in the art, *e.g.,* Southern and Northern blotting, PCR or primer extension. This embodiment may be useful for screening methods for verifying successful introduction of the nucleic acid molecules described above during gene therapy approaches.

The nucleic acid molecule(s) may be a recombinantly produced chimeric nucleic acid molecule comprising any of the aforementioned nucleic acid molecules either alone or in combination. In specific aspects, the nucleic acid molecule is part of a vector.

The present disclosure therefore also relates to a composition comprising a vector comprising the nucleic acid molecule described in the present disclosure.

Many suitable vectors are known to those skilled in molecular biology, the choice of which would depend on the function desired and include plasmids, cosmids, viruses, bacteriophages and other vectors used conventionally in genetic engineering. Methods that are well known to those skilled in the art can be used to construct various plasmids and vectors; see, for example, the techniques described in Sambrook et al. (1989) and Ausubel, Current Protocols in Molecular Biology, Green Publishing Associates and Wiley Interscience, N.Y. (1989), (1994). Alternatively, the polynucleotides and vectors of the disclosure can be reconstituted into liposomes for delivery to target cells. A cloning vector may be used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

In specific embodiments, there is a vector that comprises a nucleic acid sequence that is a regulatory sequence operably linked to the nucleic acid sequence encoding a bispecific single chain antibody constructs defined herein. Such regulatory sequences (control elements) are known to the artisan and may include a promoter, a splice cassette, translation initiation codon, translation and insertion site for introducing an insert into the vector. In specific embodiments, the nucleic acid molecule is operatively linked to said expression control sequences allowing expression in eukaryotic or prokaryotic cells.

It is envisaged that a vector is an expression vector comprising the nucleic acid molecule encoding a bispecific single chain antibody constructs defined herein. In specific aspects, the vector is a viral vector, such as a lentiviral vector. Lentiviral vectors are commercially available, including from Clontech (Mountain View, CA) or GeneCopoeia (Rockville, MD), for example.

The term "regulatory sequence" refers to DNA sequences that are necessary to effect the expression of coding sequences to which they are ligated. The nature of such control sequences differs depending upon the host organism. In prokaryotes, control sequences generally include promoters, ribosomal binding sites, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the recited vector is an expression vector, in certain embodiments. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, *e.g.,* the P_{L}, lac, trp or tac promoter in *E. coli,* and examples of regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells.

Beside elements that are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, *e.g.,* stabilization or simplified purification of expressed recombinant product; see supra. In this context, suitable expression vectors are known in the art such as Okayama-Berg cDNA expression vector pcDV1 (Pharmacia), pEF-Neo, pCDM8, pRc/CMV, pcDNA1, pcDNA3 (Invitrogen), pEF-DHFR and pEF-ADA, (Raum et al. Cancer Immunol Immunother (2001) 50(3), 141-150) or pSPORT1 (GIBCO BRL).

In some embodiments, the expression control sequences are eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the disclosure may follow.

Additional regulatory elements may include transcriptional as well as translational enhancers. Advantageously, the above-described vectors of the disclosure comprises a selectable and/or scorable marker. Selectable marker genes useful for the selection of transformed cells are well known to those skilled in the art and comprise, for example, antimetabolite resistance as the basis of selection for dhfr, which confers resistance to methotrexate (Reiss, Plant Physiol. (Life-Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used in a cell, alone, or as part of a vector to express the encoded polypeptide in cells. The nucleic acid molecules or vectors containing the DNA sequence(s) encoding any one of the above described bispecific single chain antibody constructs is introduced into the cells that in turn produce the polypeptide of interest. The recited nucleic acid molecules and vectors may be designed for direct introduction or for introduction *via* liposomes, or viral vectors (*e.g.,* adenoviral, retroviral) into a cell. In certain embodiments, the cells are T-cells, CAR T-cells, NK cells, NKT-cells, MSCs, neuronal stem cells, or hematopoietic stem cells, for example.

In accordance with the above, the present disclosure relates to methods to derive vectors, particularly plasmids, cosmids, viruses and bacteriophages used conventionally in genetic engineering that comprise a nucleic acid molecule encoding the polypeptide sequence of a bispecific single chain antibody constructs defined herein. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the recited polynucleotides or vector into targeted cell populations. Methods which are well known to those skilled in the art can be used to construct recombinant vectors; see, for example, the techniques described in Sambrook et al. (loc cit.), Ausubel (1989, loc cit.) or other standard text books. Alternatively, the recited nucleic acid molecules and vectors can be reconstituted into liposomes for delivery to target cells. The vectors containing the nucleic acid molecules of the disclosure can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

### III. VECTORS GENERALLY

The engager molecules of the present disclosure may be expressed from an expression vector. Recombinant techniques to generate such expression vectors are well known in the art.

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g*., YACs). One of skill in the art would be well equipped to construct a vector through standard recombinant techniques (see, for example, Maniatis *et al.,* 1988 and Ausubel *et al.,* 1994).

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleic acid sequences that serve other functions as well and are described infra.

### A. Promoters and Enhancers

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30 110 bp upstream of the start site, although a number of promoters have been shown to contain functional elements downstream of the start site as well. To bring a coding sequence "under the control of' a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5 prime non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.,* containing different elements of different transcriptional regulatory regions, and/or mutations that alter expression. For example, promoters that are most commonly used in recombinant DNA construction include the β lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patent Nos. 4,683,202 and 5,928,906). Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

The identity of tissue-specific promoters or elements, as well as assays to characterize their activity, is well known to those of skill in the art.

In particular embodiments, the expression of a secretable engager molecule polypeptide is modulated. The expression may be modulated in a variety of ways, although in specific embodiments one or more regulatory sequences direct expression of a polynucleotide that encodes an engager polypeptide in a spatial and/or temporal manner. In some cases, the expression is modulated to increase expression of a polynucleotide that encodes an engager polypeptide, such that there is a corresponding increase in the level of engager polypeptide in the immune cell or secreted therefrom. In some cases the expression is modulated to decrease expression of a polynucleotide that encodes an engager molecule, such that there is a corresponding decrease in the level of engager polypeptide in the immune cell or secreted therefrom. Situations where the expression may be desired to be decreased include those where the engager is undesired or no longer desired, for example in normal tissue. The modulation of expression may be compared to the level of expression in the absence of the particular regulatory sequence or factor(s) that regulates it.

In certain embodiments, the expression of an engager polypeptide is modulated upon exposure of a corresponding regulatory sequence to one or more factors. In specific embodiments, the expression is modulated upon exposure to tumor-associated factors. Illustrative examples of tumor-associated factors include factors present in hypoxic tissue. In some embodiments, the factors are cytokines and/or chemokines. For example, hypoxia induces the expresison of HIF-1α, a transcription factor that could induce engager expression that is under the control of a hypoxia response element (HRE). Hypoxia could also stabilize engager molecules that contain an oxygen-dependent degradation domain (ODDD). Another example of a substance, which is produced by tumor cells and could regulate engager gene expression, is lactic acid. A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

In certain embodiments, the use of internal ribosome entry sites (IRES) elements are used to create multigene, or polycistronic, messages, and these may be used in the embodiments.

In certain embodiments 2A sequences are used to create multigene messages, and these may be used in the embodiments.

Vectors can include a multiple cloning site (MCS), which is a nucleic acid region that contains multiple restriction enzyme sites, any of which can be used in conjunction with standard recombinant technology to digest the vector. "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

Splicing sites, termination signals, origins of replication, and selectable markers may also be employed.

### B. Plasmid Vectors

In certain aspects, a plasmid vector is contemplated for use to transform a host cell. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, E. coli is often transformed using derivatives of pBR322, a plasmid derived from an E. coli species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEMTM 11 may be utilized in making a recombinant phage vector which can be used to transform host cells, such as, for example, E. coli LE392.

Further useful plasmid vectors include pIN vectors (Inouye *et al.,* 1985); and pGEX vectors, for use in generating glutathione S transferase (GST) soluble fusion proteins for later purification and separation or cleavage. Other suitable fusion proteins are those with β galactosidase, ubiquitin, and the like.

Bacterial host cells, for example, *E. coli,* comprising the expression vector, are grown in any of a number of suitable media, for example, LB. The expression of the recombinant protein in certain vectors may be induced, as would be understood by those of skill in the art, by contacting a host cell with an agent specific for certain promoters, *e.g.,* by adding IPTG to the media or by switching incubation to a higher temperature. After culturing the bacteria for a further period, generally of between 2 and 24 h, the cells are collected by centrifugation and washed to remove residual media.

### C. Viral Vectors

The ability of certain viruses to infect cells or enter cells via receptor mediated endocytosis, and to integrate into host cell genome and express viral genes stably and efficiently have made them attractive candidates for the transfer of foreign nucleic acids into cells (*e.g*., mammalian cells). Components of the present disclosure may be a viral vector that encodes one or more CARs of the disclosure. Non-limiting examples of virus vectors that may be used to deliver a nucleic acid of the present disclosure are described below.

### i. Adenoviral Vectors

A particular method for delivery of the nucleic acid involves the use of an adenovirus expression vector. Although adenovirus vectors are known to have a low capacity for integration into genomic DNA, this feature is counterbalanced by the high efficiency of gene transfer afforded by these vectors. "Adenovirus expression vector" is meant to include those constructs containing adenovirus sequences sufficient to (a) support packaging of the construct and (b) to ultimately express a tissue or cell specific construct that has been cloned therein. Knowledge of the genetic organization or adenovirus, a 36 kb, linear, double stranded DNA virus, allows substitution of large pieces of adenoviral DNA with foreign sequences up to 7 kb (Grunhaus and Horwitz, 1992).

### ii. AAV Vectors

The nucleic acid may be introduced into the cell using adenovirus assisted transfection. Increased transfection efficiencies have been reported in cell systems using adenovirus coupled systems (Kelleher and Vos, 1994; Cotten *et al.,* 1992; Curiel, 1994). Adeno associated virus (AAV) is an attractive vector system for use in the cells of the present disclosure as it has a high frequency of integration and it can infect nondividing cells, thus making it useful for delivery of genes into mammalian cells, for example, in tissue culture (Muzyczka, 1992) or in vivo. AAV has a broad host range for infectivity (Tratschin *et al.,* 1984; Laughlin *et al.,* 1986; Lebkowski *et al.,* 1988; McLaughlin *et al.,* 1988). Details concerning the generation and use of rAAV vectors are described in U.S. Patent Nos. 5,139,941 and 4,797,368.

### iii. Retroviral Vectors

Retroviruses are useful as delivery vectors because of their ability to integrate their genes into the host genome, transferring a large amount of foreign genetic material, infecting a broad spectrum of species and cell types and of being packaged in special cell lines (Miller, 1992).

An exemplary retroviral vector comprises in a 5' to 3' direction an EphA2-specific scFv(4H5) linked to a CD3-specific scFv followed by mOrange (mO) separated by an internal ribosomal entry site (IRES) (**FIG. 4**).

In order to construct a retroviral vector, a nucleic acid (*e.g.,* one encoding the desired sequence) is inserted into the viral genome in the place of certain viral sequences to produce a virus that is replication defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into a special cell line (*e.g.,* by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

Lentiviruses are complex retroviruses, which, in addition to the common retroviral genes gag, pol, and env, contain other genes with regulatory or structural function. Lentiviral vectors are well known in the art (see, for example, Naldini *et al.,* 1996; Zufferey *et al.,* 1997; Blomer *et al.,* 1997; U.S. Pat. Nos. 6,013,516 and 5,994,136). Some examples of lentivirus include the Human Immunodeficiency Viruses: HIV-1, HIV-2 and the Simian Immunodeficiency Virus: SIV. Lentiviral vectors have been generated by multiply attenuating the HIV virulence genes, for example, the genes env, vif, vpr, vpu and nef are deleted making the vector biologically safe.

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific.

In particular aspects described herein, the retrovirus comprises an envelope proteins (env) protein that determines the range of host cells which can ultimately be infected and transformed by recombinant retroviruses generated from the cell lines. Illustrative examples of retroviral-derived env genes which can be employed in the invention include, but are not limited to: VSV-G, MLV envelopes, 10A1 envelope, BAEV, FeLV-B, RD114, SSAV, Ebola, Sendai, FPV (Fowl plague virus), gp41 and gp120, and influenza virus envelopes.

In one aspects described herein, the disclosure provides retrovirus pseudotyped with the VSV-G glycoprotein.

### 2. Other Viral Vectors

Other viral vectors may be employed as vaccine constructs in the present disclosure. Vectors derived from viruses such as vaccinia virus (Ridgeway, 1988; Baichwal and Sugden, 1986; Coupar *et al.,* 1988), sindbis virus, cytomegalovirus and herpes simplex virus may be employed. They offer several attractive features for various mammalian cells (Friedmann, 1989; Ridgeway, 1988; Baichwal and Sugden, 1986; Coupar *et al.,* 1988; Horwich *et al.,* 1990).

### D. Delivery Using Modified Viruses

A nucleic acid to be delivered may be housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes via sialoglycoprotein receptors.

Another approach to targeting of recombinant retroviruses was designed in which biotinylated antibodies against a retroviral envelope protein and against a specific cell receptor were used. The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus in vitro (Roux *et al.,* 1989).

### E. Vector Delivery and Cell Transformation

Suitable methods for nucleic acid delivery for transfection or transformation of cells are known to one of ordinary skill in the art. Such methods include, but are not limited to, direct delivery of DNA such as by *ex vivo* transfection, by injection, and so forth. Through the application of techniques known in the art, cells may be stably or transiently transformed.

### F. Ex Vivo Transformation

Methods for transfecting eukaryotic cells and tissues removed from an organism in an ex vivo setting are known to those of skill in the art. Thus, it is contemplated thaT-cells or tissues may be removed and transfected *ex vivo* using nucleic acids of the present disclosure. In particular aspects, the transplanted cells or tissues may be placed into an organism. In preferred facets, a nucleic acid is expressed in the transplanted cells.

### IV. HOST CELLS COMPRISING ENGAGERS

It is further envisaged that the pharmaceutical composition of the disclosure comprises a host cell transformed or transfected with a vector defined herein above. The host cell may be produced by introducing at least one of the above described vectors or at least one of the above described nucleic acid molecules into the host cell. The presence of the at least one vector or at least one nucleic acid molecule in the host may mediate the expression of a gene encoding the above described be specific single chain antibody constructs.

The described nucleic acid molecule or vector that is introduced in the host cell may either integrate into the genome of the host or it may be maintained extrachromosomally.

The host cell can be any prokaryote or eukaryotic cell, but in specific embodiments it is a eukaryotic cell. In specific embodiments, the host cell is a bacterium, an insect, fungal, plant or animal cell. It is particularly envisaged that the recited host may be a mammalian cell, more preferably a human cell or human cell line. Particularly preferred host cells comprise immune cells, CHO cells, COS cells, myeloma cell lines like SP2/0 or NS/0.

The pharmaceutical composition of the disclosure may also comprise a proteinaceous compound capable of providing an activation signal for immune effector cells useful for cell proliferation or cell stimulation. In particular aspects, the proteinaceous compound is not understood as an additional domain of the above defined bispecific single chain antibody construct, but at least one additional component of the pharmaceutical composition of the disclosure.

In the light of the present disclosure, the "proteinaceous compounds" providing an activation signal for immune effector cells" may be, *e.g.* a further activation signal for T-cells (*e.g.* a further costimulatory molecule: molecules of the B7-family, OX40 L, 4-1BBL), or a further cytokine: interleukin (*e.g*. IL-2, IL-7, or IL-15), or an NKG-2D engaging compound. Preferred formats of proteinaceous compounds comprise additional bispecific antibodies and fragments or derivatives thereof, *e.g.* bispecific scFv. Proteinaceous compounds can comprise, but are not limited to scFv fragments specific for the T-cell receptor or superantigens. Superantigens directly bind to certain subfamilies of T-cell receptor variable regions in an MHC-independent manner thus mediating the primary T-cell activation signal. The proteinaceous compound may also provide an activation signal for immune effector cell which is a non-T-cell. Examples for immune effector cells which are non-T-cells comprise, *inter alia,* NK cells, or NKT-cells.

One aspect described herein relates to a process for the production of a composition of the disclosure, the process comprising culturing a host cell defined herein above under conditions allowing the expression of the construct and recovering the produced bispecific single chain antibody construct from the culture. However, in particular aspects, the cell or a plurality of cells is provided to the individual.

The conditions for the culturing of cells harboring an expression construct that allows the expression of the engager molecules are known in the art, as are procedures for the purification/recovery of the constructs when desired.

In one aspect, the host cell is a T-cell comprising an engineered TCR receptor or a CAR. Naturally occurring T-cell receptors comprise two subunits, an α-subunit and a β-subunit, each of which is a unique protein produced by recombination event in each T-cell's genome. Libraries of TCRs may be screened for their selectivity to particular target antigens. An "engineered TCR" refers to a natural TCR, which has a high-avidity and reactivity toward target antigens that is selected, cloned, and/or subsequently introduced into a population of T-cells used for adoptive immunotherapy. In contrast to engineered TCRs, CARs are engineered to bind target antigens in an MHC independent manner. In particular aspects, a CAR comprises an extracellular binding domain including, but not limited to, an antibody or antigen binding fragment thereof; a transmembrane domain; one or more intracellular costimulatory signaling domains and a primary signaling domain.

In various aspects described herein, a T-cell comprises one or more polynucleotides encoding engager molecules that recognize the same target antigen as a CAR or engineered TCR expressed by the T-cell. In particular embodiments, a CAR or engineered TCR expressing T-cell comprises one or more polynucleotides encoding engager molecules that recognize a target antigen that is different than the target antigen recognized by a CAR or engineered TCR, but that is expressed on the same target cell.

### V. PHARMACEUTICAL COMPOSITIONS

In accordance with this disclosure, the term "pharmaceutical composition" relates to a composition for administration to an individual. In a preferred aspect, the pharmaceutical composition comprises a composition for parenteral, transdermal, intraluminal, intra-arterial, intrathecal or intravenous administration or for direct injection into a cancer. It is in particular envisaged that said pharmaceutical composition is administered to the individual *via* infusion or injection. Administration of the suitable compositions may be effected by different ways, *e.g.,* by intravenous, subcutaneous, intraperitoneal, intramuscular, topical or intradermal administration.

The pharmaceutical composition of the present disclosure may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, *etc.* Compositions comprising such carriers can be formulated by well known conventional methods. These pharmaceutical compositions can be administered to the subject at a suitable dose.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A preferred dosage for administration might be in the range of 0.24 µg to 48 mg, preferably 0.24 µg to 24 mg, more preferably 0.24 µg to 2.4 mg, even more preferably 0.24 µg to 1.2 mg and most preferably 0.24 µg to 240 mg units per kilogram of body weight per day. Particularly preferred dosages are recited herein below. Progress can be monitored by periodic assessment. Dosages will vary but a preferred dosage for intravenous administration of DNA is from approximately 10⁶ to 10¹² copies of the DNA molecule.

The compositions of the disclosure may be administered locally or systemically. Administration will generally be parenteral, e.g., intravenous; DNA may also be administered directly to the target site, *e.g.,* by biolistic delivery to an internal or external target site or by catheter to a site in an artery. In a preferred aspect, the pharmaceutical composition is administered subcutaneously and in an even more preferred embodiment intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present disclosure might comprise proteinaceous carriers, like, *e.g.,* serum albumin or immunoglobulin, preferably of human origin. It is envisaged that the pharmaceutical composition of the disclosure might comprise, in addition to the proteinaceous bispecific single chain antibody constructs or nucleic acid molecules or vectors encoding the same (as described in this disclosure), further biologically active agents, depending on the intended use of the pharmaceutical composition.

Any of the compositions described herein may be comprised in a kit. In a non-limiting example, one or more cells for use in cell therapy and/or the reagents to generate one or more cells for use in cell therapy that harbors recombinant expression vectors may be comprised in a kit. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly ueful. In some cases, the container means may itself be a syringe, pipette, and/or other such like apparatus, from which the formulation may be applied to an infected area of the body, injected into an animal, and/or even applied to and/or mixed with the other components of the kit.

However, the components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile, pharmaceutically acceptable buffer and/or other diluent.

In particular aspects described herein, cells that are to be used for cell therapy are provided in a kit, and in some cases the cells are essentially the sole component of the kit. The kit may comprise reagents and materials to make the desired cell. In specific aspects, the reagents and materials include primers for amplifying desired sequences, nucleotides, suitable buffers or buffer reagents, salt, and so forth, and in some cases the reagents include vectors and/or DNA that encodes an engager molecule as described herein and/or regulatory elements therefor.

In particular aspects described herein, there are one or more apparatuses in the kit suitable for extracting one or more samples from an individual. The apparatus may be a syringe, scalpel, and so forth.

In some cases, the kit, in addition to cell therapy aspects, also includes a second cancer therapy, such as chemotherapy, hormone therapy, and/or immunotherapy, for example. The kit(s) may be tailored to a particular cancer for an individual and comprise respective second cancer therapies for the individual.

### VI. THERAPEUTIC USES OF ENGAGERS AND HOST T-CELLS COMPRISING ENGAGERS

In various apects bispecific single chain antibody constructs, nucleic acid sequences, vectors, host cells, as contemplated herein and/or pharmaceutical compositions comprising the same are used for the prevention, treatment or amelioration of a cancerous disease, such as a tumorous disease. In particular aspects described herein, the pharmaceutical composition of the present disclosure may be particularly useful in preventing, ameliorating and/or treating cancer, including cancer having solid tumors, for example.

As used herein "treatment" or "treating," includes any beneficial or desirable effect on the symptoms or pathology of a disease or pathological condition, and may include even minimal reductions in one or more measurable markers of the disease or condition being treated, *e.g.,* cancer. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" *etc.,* indicate an approach for preventing, inhibiting, or reducing the likelihood of the occurrence or recurrence of, a disease or condition, *e.g.,* cancer. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In particular aspects described herein, the present disclosure contemplates, in part, cells, bispecific single chain antibody construct, nucleic acid molecules and vectors that can administered either alone or in any combination using standard vectors and/or gene delivery systems, and in at least some aspects, together with a pharmaceutically acceptable carrier or excipient. In certain aspects, subsequent to administration, said nucleic acid molecules or vectors may be stably integrated into the genome of the subject.

In specific aspects, viral vectors may be used that are specific for certain cells or tissues and persist in said cells. Suitable pharmaceutical carriers and excipients are well known in the art. The compositions prepared according to the disclosure can be used for the prevention or treatment or delaying the above identified diseases.

Furthermore, the disclosure relates to a method for the prevention, treatment or amelioration of a tumorous disease comprising the step of administering to a subject in the need thereof an effective amount of cells harboring an engager molecule, a nucleic acid sequence, a vector, as contemplated herein and/or produced by a process as contemplated herein.

Possible indications for administration of the composition(s) of the exemplary EphA2 Engager cells are cancerous diseases, including tumorous diseases, including breast, prostate, lung, and colon cancers or epithelial cancers/carcinomas such as breast cancer, colon cancer, prostate cancer, head and neck cancer, skin cancer, cancers of the genito-urinary tract, *e.g*. ovarian cancer, endometrial cancer, cervix cancer and kidney cancer, lung cancer, gastric cancer, cancer of the small intestine, liver cancer, pancreas cancer, gall bladder cancer, cancers of the bile duct, esophagus cancer, cancer of the salivary glands and cancer of the thyroid gland. In particular aspects, the cancer is EphA2-positive, for example. Exemplary indications for administration of the composition(s) of CD19 Engager cells are cancerous diseases, including any malignancies that express CD19. These include in general all hematological malignancies that are derived from the B-cell lineage. In addition, it includes malignancies that aberrantly express CD19. The administration of the composition(s) of the disclosure is useful for all stages and types of cancer, including for minimal residual disease, early cancer, advanced cancer, and/or metastatic cancer and/or refractory cancer, for example.

The disclosure further encompasses co-administration protocols with other compounds, *e.g.* bispecific antibody constructs, targeted toxins or other compounds, which act *via* immune cells. The clinical regimen for co-administration of the inventive compound(s) may encompass co-administration at the same time, before or after the administration of the other component. Particular combination therapies include chemotherapy, radiation, surgery, hormone therapy, or other types of immunotherapy.

Aspects described herein relate to a kit comprising a bispecific single chain antibody construct as defined above, a nucleic acid sequence as defined above, a vector as defined above and/or a host as defined above. It is also contemplated that the kit of this disclosure comprises a pharmaceutical composition as described herein above, either alone or in combination with further medicaments to be administered to an individual in need of medical treatment or intervention.

### VII. COMBINATION THERAPY

In certain aspects, methods of the present disclosure for clinical aspects are combined with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents, for example, represents a major problem in clinical oncology. One goal of current cancer research is to find ways to improve the efficacy of chemo- and radiotherapy by combining it with other therapies. In the context of the present disclosure, it is contemplated that T-cell therapy could be used similarly in conjunction with chemotherapeutic, radiotherapeutic, or immunotherapeutic intervention, as well as pro-apoptotic or cell cycle regulating agents.

Alternatively, the present inventive therapy may precede or follow the other agent treatment by intervals ranging from minutes to weeks. In aspects where the other agent and present disclosure are applied separately to the individual, one would generally ensure that a significant period of time did not expire between the time of each delivery, such that the agent and inventive therapy would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

Various combinations may be employed, present disclosure is "A" and the secondary agent, such as radio- or chemotherapy, for example, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

It is expected that the treatment cycles would be repeated as necessary. It also is contemplated that various standard therapies, as well as surgical intervention, may be applied in combination with the inventive cell therapy.

### A. Chemotherapy

Cancer therapies also include a variety of combination therapies with both chemical, radiation-based treatments, and/or non-immune based trageted therapies. Combination chemotherapies include all classes of chemotherapeutic agents including alkylating agents, antimetabalites, plant alkaloids, antibiotics, hormonal agents, and miscellaneous anticancer drugs. Specific agents include, for example, abraxane, altretamine, docetaxel, herceptin, methotrexate, novantrone, zoladex, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabine, fuldarabine, navelbine, farnesyl-protein tansferase inhibitors, transplatinum, 5-fluorouracil, vincristin, and vinblastin, or any analog or derivative variant of the foregoing and also combinations thereof.

In specific embodiments, chemotherapy for the individual is employed in conjunction with the disclosure, for example before, during and/or after administration of the embodiments.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Non-immune based targeted therapies

Cancer therapies also include a variety of combination therapies with non-immune based targeted therapies. These include for example agents that inhibit signaling pathways such WNT, p53, and/or RB-signaling pathways. Other examples include agents that inhibit tyrosine kinases, BRAF, STAT3, c-met, regulate gene expression, induce cell death or block blood vessel formation. Examples of specific agents include imatinib mesylate, dasatinib, nilotinib, bosutinib, lapatinib, gefinitib, erlotinib, tensirolimus, everolimus, vemurafenib, crizotinib, vorinostat, romidepsin, bexarotene, alitrionin, tretionin, bortezomib, carfilzomib, pralatrexate, sorafenib, sunitinib, pazopanib, regorafenib, or cabozantinib.

### D. Immunotherapy

Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effecT-cell killing. The antibody also may be conjugated to a drug or toxin (chemotherapeutic, radionuclide, ricin A chain, cholera toxin, pertussis toxin, *etc*.) and serve merely as a targeting agent. Alternatively, the effector may be a lymphocyte carrying a surface molecule that interacts, either directly or indirectly, with a tumor cell target. Various effector cells include cytotoxic T-cells and NK cells.

Immunotherapy other than the inventive therapy described herein could thus be used as part of a combined therapy, in conjunction with the presenT-cell therapy. The general approach for combined therapy is discussed below. Generally, the tumor cell must bear some marker that is amenable to targeting, *i.e*., is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155 and the like.

### E. Genes

In yet another embodiment, the secondary treatment is a gene therapy in which a therapeutic polynucleotide is administered before, after, or at the same time as the present disclosure clinical embodiments. A variety of expression products are encompassed within the disclosure, including inducers of cellular proliferation, inhibitors of cellular proliferation, or regulators of programmed cell death.

### F. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present disclosure, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present disclosure may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### G. Other agents

It is contemplated that other agents may be used in combination with the present disclosure to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abililties of the present disclosure by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present disclosure to improve the anti-hyerproliferative efficacy of the treatments. Inhibitors of cell adhesion are contemplated to improve the efficacy of the present disclosure. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present disclosure to improve the treatment efficacy.

### EXAMPLES

The following examples are presented in order to more fully illustrate the preferred embodiments of the disclosure. They should in no way, however, be construed as limiting the broad scope of the disclosure.

### VIII. EXAMPLE 1: GENERATION OF ENGAGER CELLS

The inventors have developed a new class of cells (for example, but not limited to T-cells, NK, or NKT-cells) by genetically modifying them with secretable molecules, termed engagers. Engagers comprise an antigen recognition domain and an activation domain. Antigen recognition and activation domains can bind single or multiple molecules and comprises, for example, of 1) scFvs, 2) peptides, and/or 3) natural ligands. The antigen recognition domain binds to molecules that are present in and/or on target cells or are secreted by target cells. The activation domain recognizes molecules expressed on the cell surface of cells or molecules that are secreted by cells. Example of activation domains include domains that bind to CD3, CD16, CD28, CD40, CD134, or CD137. The exemplary mode of action of engager cells is summarized in FIGS. 1 and 3.

Provided herein are exemplary T-cells that secrete engagers comprising, as an example, an antigen recognition domain specific for EphA2, CD19. CD123, LeY, B7H3, HER2, or EGFR and an activation domain specific for CD3 or CD16 using retroviral vectors (EphA2 T-cell engager (**FIG. 6**), CD19 T-cell engager (**FIG. 7**), CD123 T-cell engager (**FIG. 8**), LeY T-cell engager (**FIG. 9**), B7H3 T-cell engager (**FIG. 10**), HER2 T-cell engager (**FIG. 11**), or EGFR T-cell engager (**FIG. 12**), and EphA2 NK-cell engager (**FIG. 13**).

### IX. EXAMPLE 2: ENGAGER T-CELLS RECOGNIZE TARGET CELLS IN AN ANTIGEN DEPENDENT MANNER

In particular aspects, the inventors provide an alternative strategy to render T-cells specific for antigens that utilizes expressing a secretable bispecific T-cell engager in T-cells that comprises two scFVs. One scFV is specific for CD3 and the other scFV is specific for an antigen of choice. The inventors constructed 1) a bispecific T-cell engager that recognizes CD3 and the tumor antigen EphA2; 2) a bispecific T-cell engager that recognizes CD3 and the tumor antigen CD19; and 3) a bispecific T-cell engager that recognizes CD3 and the tumor antigen CD123, 4) a bispecific T-cell engager that recognizes CD3 and the tumor antigen LeY, 5) a bispecific T-cell engager that recognizes CD3 and the tumor antigen B7H3, 6) a bispecific T-cell engager that recognizes CD3 and the tumor antigen HER2, and 7) a bispecific T-cell engager that recognizes CD3 and the tumor antigen EGFR. Engager T-cells were generated by viral transduction of T-cells as an exemplary method. Because engager molecules cannot be readily detected, an EphA2-specific engager molecule was generated with a 6xHis-myc tag (EphA2-HM ENG; **FIG. 5A**). T-cells expressing standard EphA2-ENG and EphA2-HM ENG killed EphA2-positive target cells, demonstrating that EphA2-HM ENG is functional (**FIG. 5B**). Using myc antibodies and 6xHis antibodies, it was demonstrated that EphA2-HM ENG molecules bind to the cell surface of T-cells (**FIG. 5C**) and are secreted into the media (**FIG. 5D**). Part of this embodiment is that a 3^{rd} domain can be added to the engager molecule to enhance it function. Here there has been added an 'recognition tag', demonstrating that it is feasible to further modify engager molecules.

**EphA2-ENG T-cells recognize EphA2-positive tumor cells.** T-cells expressing the CD3/EphA2 T-cell engager (EphA2-ENG-T-cells) recognize EphA2-positive cancer cells (**FIG. 6A**). EphA2-ENG-T-cells were co-cultured with EphA2-positive (U373, A549) and EphA2-negative (K562) tumor cells. After 24 hours, media was collected for analysis. EphA2-ENG-T-cells recognized U373 and A549 in contrast to K562 as judged by the production of the proinflammatory cytokine IFNγ. K562 cells genetically modified to express EphA2 induced IFNγ production, highlighting that EphA2 has to be expressed by target cells to induce the production IFNγ. Non-transduced (NT) T-cells cells and T-cells secreting an engager that recognizes an antigen not expressed on these tumor cells (CD19) produced no IFNγ. EphA2-ENG-T-cells also proliferated in the presence of EphA2-positive tumor cells in contrast to NT and CD19-specific T-cells. Results for 4 exemplary donors are shown (**FIG. 6B**).

**CD19-ENG-T-cells kill CD19-positive tumor cells** (**FIG. 7**). CD19 Engager T-cells were generated by retroviral transduction and ∼ 50% of T-cells were transduced as judged by FACS analysis (**FIG. 7A****,B**). CD19-ENG T-cells recognized CD19-positive target cells as judged by chromium release assay (**FIG. 7C**), and IFN-γ and IL-2 secretion (**FIG. 7D**) in contrast to CD19-negative K562 cells. None of the targets were recognized by non-transduced (NT) T-cells or T-cells secreting engagers specific for an irrelevant antigen (EphA2-ENG T-cells).

**CD123-ENG T-cells kill CD123-positive tumor cells** (**FIG. 8**). Two retroviral vectors were generated encoding CD123-specific engager molecules derived from the CD123-specific MAbs 26292 (292) and 32716 (716), which bind to distinct epitopes on the CD123 molecule (**FIG. 8A**). CD123(292)- and CD123(716)-specific T-cells were generated by retroviral transduction and greater 80% of T-cells were genetically modified as judged by FACS analysis (**FIG. 8A**). CD123(292)- and CD123(716)-specific ENG T-cells recognized CD123-positive target cells, KG1a and JurkaT-cells that were genetically modified to express CD123 (Jurkat-CD123), in co-culture assays as judged by IFNγ secretion (**FIG. 8B****,C**). In contrast CD123-negative, parental JurkaT-cells did not induce IPNγ secretion, and ENG T-cells specific for an irrelevant antigen (CD19) did not release cytokines in response to any of the tested target cells (**FIG. 8C**). In a standard cytotoxicity assay, CD123(292)- and CD123(716)- specific ENG T-cells killed KG1a cells in contrast of CD19-specific ENG T-cells, confirming antigen specificity (**FIG. 8D**).

**LeY-ENG T-cells kill LeY-positive tumor cells** (**FIG. 9**). To demonstrate that LeY-ENG T-cells kill LeY-positive target cells we performed standard cytotoxicity assyswith LeY-ENG T-cells and CD19-ENG T-cells as effectors and K562 (CD19-, LeY-) and KG1a (CD19-, LeY+) target cells. Only LeY-positive target cells were killed by LeY-ENG T-cells. In contrast CD19-ENG T-cells had no cytolytic activity.

**B7H3-ENG T-cells recognize and kill B7H3-positive tumor cells** (**FIG. 10**). To determine if B7H3-ENG T-cells specifically recognzie B7H3-postive tumor cells, we performed co-culture assay of B7H3-ENG T-cells with B7H3-positive (U373, LM7, CHLA255) and B7H3-negative (HTB119) tumor cells. CD19-ENG T-cells served as controls since all tested tumor cells did not express CD19. Only B7H3-positive trageT-cells induced IFNγ production of B7H3-ENG T-cells (**FIG. 10A**) demonstrating antigen-specific activation of B7H3-ENG T-cells. To determine the cytolytic activity of B7H3-ENG T-cells U373, LM7, CHLA255 were incubated with B7H3-ENG T-cells or CD19-ENG T-cells. While B7H3-ENG T-cells killed all tumor cells as judged by crystal violet staining, no killing was observed in the presence of CD19-ENG T-cells (**FIG. 10B**)**.**

**HER2-ENG T-cells recognize HER2-positive tumor cells** (**FIG. 11**). To demonstrate that HER2-ENG T-cells recognize HER2-positive target cells in an antigen depdendent manner, we co-cultured HER2-ENG T-cells or non-transduced (NT) T-cells were co-cultured with HER2-positive (U373) and HER2-negative (MDA) tumor cells. After 24 hours IPNγ was determined. While U373 induced IFNγ production MDA did not, demonstrating antigen-specific recognition of HER2-positive tumor cells. No IFNγ production was observed with either targets in the presence of NT T-cells.

**806-ENG T-cells recognize EGFR-positive tumor cells** (**FIG. 12**). To demonstrate that 806-ENG T-cells recognize the conformatinal EGFR epitope 806 in cells in which EGFR is gene amplified or that express EGFRvIII we performed co-culture assays with U373 (EGFR low positive), A431(EGFR gene amplified), K562 (EGFR negative), and K562 genetically modified to express EGFRvIII (K562-EGFRvIII). Significant IFNγ production was observed in the presence of A431 and K562-EGFRvIII. In contrast none of the targets (all CD19-negative) induced IFNg production of CD19-ENG T-cells.

**T-cells secreting EphA2-specific NK-cell engagers activate NK cells in an antigen-specific manner** (**FIG. 13**). T-cell were transduced with a retroviral vertor encoding a NK-cell engager consisting of a CD16-specific scFv linked to an EphA2-specific scFv (**FIG 13A**). Transduced T-cells (CD16.EphA2-ENG T-cells) were incubated on IL13Rα2- or EphA2-coated plates in the absence or presence of autologous NK cells. IFNγ production was measured after 24 hours. CD16.EphA2-ENG T-cells/NK cells cocultures produced high levels of IFNy in the presence of EphA2 but not in the presence of IL13Ra2. In addition, CD16.EphA2-ENG T-cells or NK cells by themselves did not produce IFNγ indicating that CD16.EphA2-ENG Tcells are able to redirect NK cells specifically to EphA2 (**FIG 13B**).

### X. EXAMPLE 3: ENGAGER T-CELLS REDIRECT BYSTANDER T-CELLS TO TARGET CELLS - IN VITRO STUDIES

**Supernatants of EphA2-ENG-T-cells 'arm' non-transduced T-cells to recognize tumor cells** (FIG. 14A). Media was collected from EphA2-ENG-T-cells and mixed with non-transduced T-cells and tumor cells. Non-transduced T-cells produced IFNγ after exposure to U373, indicative of T-cell activation. In contrast, non-transduced T-cells did not produce IFNγ when mixed with media harvested from non-transduced T-cells. Thus, EphA2-ENG-T-cells secrete T-cell engangers to 'arm' bystander T-cells. Results for 4 exemplary donors are shown.

**EphA2-ENG-T-cells 'arm' non-transduced T-cells to recognize tumor cells** (FIG. 14B,C). EphA2-ENG-T-cells were plated in the transwell of a coculture assay and tumor cells, and non-transduced T-cells were plated in the bottom (platewell). Viable tumor cells were determined by crystal-violett staining. Tumor cell killing dependent on the presence of non-transduced T-cells, demonstrating that EphA2-ENG T-cells actively secrete Engagers. CD19-ENG T-cells had no anti-tumor effect, highlighting again the specificity of the approach.**Direct comparison of EphA2-ENG T-cells and EphA2-CAR T-cells (****FIG. 14D****)**. The activity was compared of T-cells expressing a 2^{nd} generation CAR containing the same EphA2-specific scFv as the engager (EphA2-CAR T-cells). U373 cells were incubated with 1x10⁵ T-cells containing increasing percentages of transduced EphA2-ENG or EphA2-CAR T-cells. After 48 hours viable tumor cells were measured by MTS assay. To achieve greater 99% tumor cell killing, only ∼10% of T-cells had to express EphA2 engagers. The same anti-tumor activity was only observed when ∼75% of T-cells expressed EphA2-CARs (p<0.00001).

**CD19-ENG-T-cells 'arm' non-transduced T-cells to recognize tumor cells (****FIG. 15****).** EphA2-ENG-T-cells were plated in the transwell of a coculture assay and luciferase expressing BV173 tumor cells, and non-transduced T-cells were plated in the bottom (platewell). Viable tumor cells were determined by luciferase assay. Tumor cell killing dependent on the presence of non-transduced T-cells, demonstrating that CD19-ENG T-cells actively secrete Engagers. EphA2-ENG T-cells had no anti-tumor effect, highlighting again the specificity of the approach.

**EphA2-ENG-T-cells secrete more Engagers upon activation (****FIG.16****)**. EphA2-ENG-T-cells were activated with EphA2 protein or control (HER2) protein. Activated EphA2-ENG T-cells secreted IFNγ (**FIG. 16A**) and more engager molecules (**FIG. 16B**). The antitumor activity of activated and non-activated Engager T-cells was evaluated in a transwell coculture assay with luciferase-expressing, EphA2-positive U373 cells. EphA2-activated EphA2-ENG T-cells were ∼10-fold more potent than control EphA2-ENG T-cells, demonstrating that more engagers are secreted upon T-cell activation (**FIG. 16C**). No increase in killing of activated EphA2-ENG T-cells was observed against EphA2-negative tumor cells (BV173) confirming specificity (**FIG. 16D**).

### XI. EXAMPLE 4: ENGAGER T-CELLS REDIRECT BYSTANDER T-CELLS TO TARGET CELLS - IN VIVO STUDIES

**EphA2-engagers induce the expansion of transduced and bystander T-cells *in vivo* (****FIG. 17****).** The human A549 lung cancer SCID xenograft model was used to demonstrate that EphA2-ENG T-cells expand *in vivo.* Tumor-bearing (n=5) or control (n=5) mice were injected intravenously (i.v.) with an admixture of 5x10⁶ eGFP.ffluc-expressing EphA2-ENG T-cells and 5x10⁶ unmodified T-cells, and received one intraperitoneal (i.p.) dose of IL2. While EphA2-ENG T-cells expanded in tumor-bearing mice, no expansion was observed in the absence of tumors (**FIG. 17A**). To demonstrate that EphA2-ENG T-cells induce the expansion of bystander T-cells *in vivo,* tumor-bearing mice were injected i.v. with an admixture of 5x10⁶ EphA2-ENG T-cells and 5x10⁶ eGFP.ffLuc expressing T-cells (n=5) or 5x10⁶ CD19-ENG T-cells and 5x10⁶ eGFP.ffLuc expressing T-cells (n=5). eGFP.ffLuc-expressing T-cells only expanded when co-injected with EphA2-ENG T-cells as judged by bioluminescence imaging (**FIG. 17B**). These results indicate that EphA2-engagers induced the expansion of transduced and bystander T-cells in an antigen-dependent manner *in vivo.*

### XII. EXAMPLE 5: ENGAGER T-CELLS HAVE POTENT ANTITUMOR ACTIVITY IN VIVO

The *in vivo* antitumor activity of Engager T-cells was evaluated in 4 animal models. The experimental schema are summarized in **FIG. 18****.**

**EphA2-ENG T-cells have potent antitumor activity in glioma model (****FIG. 19A,C****).** The antitumor activity of EphA2-ENG T-cells in human glioma and lung cancer SCID xenograft models was evaluated. Seven days after intracranial injection of 1x10⁵ U373.eGFP.ffLuc cells, mice were stereotactically injected at the tumor site with 2x10⁶ EphA2-ENG T-cells (n=8), or CD19-ENG T-cells (n=5). Untreated animals served as controls (n=5). Serial bioluminescence imaging was used to track tumor growth (**FIG. 19A****,B**). Mice treated with EphA2-ENG T-cells had a 2 log or greater reduction in their tumor signal, resulting in a long-term tumor free survival of 5 out of 8 mice (p<0.0005) (**FIG. 19C**).

**EphA2-ENG T-cells have potent antitumor activity in systemic lung cancer model (****FIG. 19D,F****).** The antitumor efficacy of EphA2-ENG T-cells in the A549.eGFP.ffLuc metastatic lung cancer model was evaluated. 2.5x10⁶ A549.eGFP.ffLuc cells were injected i.v. on day 0, and on day 7, 14, 21 mice received 1x10⁷ EphA2-ENG T-cells (n=5) or CD19-ENG T-cells (n=4) i.v. with one i.p. dose of IL2. Untreated animals served as controls (n=5). Only mice treated with EphA2-ENG T-cells had a significant reduction (p<0.005) in their tumor signal as early as 5 days post the 1^{st} T-cell dose (**Fig. 19D****,E**), resulting in a survival advantage in comparison to untreated mice and mice treated with CD19-Engager T-cells (p<0.005) (**Fig. 19F**).

**CD19-ENG T-cells have potent antitumor leukemia model (****FIG. 20A,B****).** The antitumor activity of CD19-ENG T-cells in the BV173.ffLuc NSG leukemia model was determined. BV173.ffLuc cells were injected i.v. on day 0, and on day 7, 14, 21 mice received 1x10⁷ CD-19 ENG T-cells (n=5) or EphA2-ENG T-cells (n=5) i.v. with one i.p. dose of IL2. Untreated animals served as controls (n=5). Only mice treated with CD19-ENG T-cells had a significant reduction (p<0.005) in their tumor signal. All mice treated with CD19-ENG T-cells were cured from their disease in contrast to mice, which received EphA2-ENG T-cells.

**CD19-ENG T-cells have potent antitumor lymphoma model (****FIG. 21A,B****).** Daudi.ffLuc cells were injected i.v. on day 0, and on day 3, 6, 9 mice received 1x10⁷ CD-19 ENG T-cells (n=5) or non-transduced (NT) T-cells (n=5) i.v. While in mice treated with NT-T-cells tumors grew exponentially, no growth was observed in CD19-ENG T-cells treated mice.

### XIII. EXAMPLE 6: THE FUNCTION OF ENGAGER T-CELLS CAN BE ENHANCED BY EXPRESSING CO-STIMULATORY MOLECULES ON THE CELL SURFACE OF T-CELLS OR IL15

**Generation of CD19-ENG T-cells that coexpress co-stimulatory molecules (****FIG. 22****)**. Co-stimulation can be provided by expressing co-stimulatory molecules on the T-cell surface. Once T-cells get activated, they express the corresponding ligands, resulting in sustained T-cell activation. A retroviral vector encoding 41BBL and CD80 separated by an IRES (**FIG. 22A**) was generated. 'Double' transduction of T-cells with retroviral vectors encoding engager molecules or CD80 and 41BBL resulted in the expression of CD80 and 41BBL on the cell surface of T-cells in contrast to T-cells that where only transduced with the retrovirus encoding the engager molecule (**FIG. 22B**). There was consistent IL2 production in the presence of target cells that do not express co-stimulatory molecules (**FIG. 22B**), highlighting that it is feasible to introduce additional genetic modification into T-cells to enhance their function.

**Generation of T-cells that express EphA2-ENG and IL15 (****FIGS. 23** **and** **24****)**. EphA2-ENG/IL15 T cells were generated by 'double' transduction of T-cells with retroviral vectors encoding engager molecules or IL15. Post stimulation with EphA2-positive tumor cells, EphA2-ENG/IL15 T cells produced IFNγ, IL2, and IL15 (**FIG. 23**). There was increased proliferation of EphA2-ENG/IL15 T cells stimulation in comparison to T cells that only express EphA2-ENG (**FIG. 24**), highlighting that it is feasible to introduce additional genes, besides genes that encode co-stimulatory molecules, into T-cells to enhance their function.

### XIV. EXAMPLE 7: SUMMARY OF CERTAIN EMBODIMENTS

Herein is described the development and characterization of a new class of T-cells that secrete bispecific T-cell engagers, and it is shown that T-cells secreting antigen-specific engagers effectively target antigen-positive cells. These engager T-cells produce immunostimulatory cytokines and proliferate in an antigen-specific manner, induce tumor cytolysis when co-cultured with antigen-positive targets, redirect bystander T-cells to antigen-positive tumor cells, and have potent antitumor activity *in vivo.*

Genetic modification of T-cells with CARs or engineered TCRs is an attractive strategy to rapidly generate antigen-specific T-cells. However, neither CAR nor engineered TCR T-cells have been shown to be able to redirect bystander T-cells to cancer cells. Several groups of investigators have developed bispecific antibodies, including bispecific T-cell engagers (BiTEs), dual affinity re-targeting antibodies (DARTs), and diabodies, to redirect resident T-cells to tumor cells. Among these, the CD19-specific BiTE, blinatumomab, has shown encouraging results in Phase I and II clinical studies for patients with hematological malignancies. However, BiTEs have to be given as a continuous infusion, which can be associated with systemic toxicities. In addition, like regular MAbs, BiTEs lack active biodistribution or self amplify once infused. In addition, they do not penetrate tissue planes, which might explain the so far limited activity of BiTEs in humans with solid tumors.

T-cells secreting engager molecules can overcome many limitations of bispecific MAbs since they are able to persist and expand post infusion, actively traffic to tumor sites, and increase transgene expression upon activation. Indeed, engager T-cells expanded *in vivo*, obviating the need for continuous infusion of engager molecules. Once activated, engager cells increased the production of the engager molecules resulting in an enhanced ability to redirect bystander T-cells to tumor cells. In particular aspects, these favorable characteristic of T-cells result in high concentrations of engager molecules at tumor sites while minimizing systemic exposure, which can be toxic.

*In vivo*, engager T-cells had potent antitumor activity in 4 animal models, demonstrating their great therapeutic potential.

In conclusion, engager T-cells present a new class of antigen-specific T-cells with the unique ability to redirect bystander T-cells to tumor cells in an antigen-dependent manner. Engager T-cells induced the regression of established tumors in locoregional and systemic xenografts models, and thus are useful to improve current immunotherapy for cancer.

### XV. EXAMPLE 8: MATERIALS AND METHODS

The present examples describes specific but exemplary aspects related to EphA2. The skilled artisan recognizes that such examples are extrapolatable to other embodiments and well within the routine skill of the artisan.

### A. Tumor cell lines

The lung cancer cell line A549, leukemia cell line K562, and glioblastoma line U373 were purchased from the American Type Culture Collection (ATCC). The leukaemia cell line BV173 was purchased from the Leibniz Institute DSMZ - German Collection of Microoganisms and Cell Cultures (Braunschweig, Germany). The generation of K562 cells expressing human EphA2 (K562-EphA2) and ffLuc-expressing U373, A549 and BV173 cells were described previously.

### B. Construction of retroviral vectors encoding EphA2-specific and CD19-specific ENGs

The construction of the EphA2-specific engager containing the immunoglobulin heavy-chain leader peptide, the EphA2-specific scFv 4H5, a short serine-glycine linker, and a CD3-specific scFV derived from OKT3 is described elsewhere. The EphA2-specific Engager was subcloned into pSFG-IRES-mOrange. The CD19-specific engager, containing the immunoglobulin heavy-chain leader peptide, the CD19-specific scFv (FMC63), a short serine-glycine linker, and a CD3-specific scFV derived from OKT3 was synthesized by Invitrogen (Carlsbad, CA) and subcloned into pSFG-IRES-mOrange. RD114-pseudotyped retroviral particles were generated.

### C. Generation of ENG T-cells

ENG- or CAR-expressing T-cells were generated as previously described. Prior to blood collection, informed consent was obtained from healthy donors in accordance to protocols approved by the Institutional Review Board of Baylor College of Medicine. PBMCs were stimulated on OKT3 and CD28 antibodies-coated non-tissue culture treated 24-well plates. Human IL2 (Proleukin, Chiron) was added to cultures on day 2, and on day 3 T-cells were transduced with retroviral particles on RetroNectin (Clontech) coated plates in the presence IL2. T-cells were subsequently expanded with IL2. NT T-cells were activated with OKT3/CD28 and expanded in parallel with IL2.

### D. Flow cytometry

The expression of mOrange was detected by FACS analysis. The surface expression of CAR on T-cells was analyzed using a CH2CH3 Cy5 antibody (Jackson ImmunoResearch Laboratories). For immunophenotyping, cells were stained with CD3-PerCP, CD4-FITC, and CD8-FITC monoclonal antibodies (BD Biosciences). Isotype controls were immunoglobulin G1-fluorescein isothiocyanate (IgG1-FITC, BD Biosciences), IgG1-peridinin chlorophyll protein (IgG1-PerCP, BD Biosciences), and isotype Cy5 (Jackson ImmunoResearch Laboratories). For each sample, 20,000 cells were analyzed by a FACSCalibur instrument (BD Biosciences) using Cell Quest Software (BD Biosciences).

### E. Ex vivo functional analysis of T-cells

EphA2-ENG, CD19-ENG, and NT T-cells were plated at 10:1 ratio with tumor cells. IPNγ and IL2 produciton after 24 hr of coculture was measured using ELISA as per the manufacturer's instructions (R&D Systems). Standard chromium (⁵¹Cr) release assays were performed as previously described.

### F. Transwell assay

U373, U373.eGFP.ffLuc or BV173.ffLuc cells were plated on bottom wells of 24 well plate. After 24 hours, NT T-cells were added to bottom wells and EphA2-ENG or CD19-ENG T-cells were added to transwell insert wells (6.5 mm in diameter, 0.4 µm pore, polycarbonate, Corning Inc). After 48 hours, viable tumor cells were detected by crystal violet staining for U373 or by luciferase assay for U373.eGFP.ffLuc or BV173.ffLuc cells.

### G. MTS assay

U373 cells were plated in 96 well plates at a density of 1x10⁴ cells per well. After 24 hours, T-cells were added to plates. After 48 hours co-culture, nonadherenT-cells were removed and viable cells were detected by 3-(4,5- dimethylthiazol-2-yl)-5-(3-carboxymethoxyphenyl)-2-(4-sulfophenyl)-2H-tetrazolium (MTS) assay (CellTiter 96 aqueous one solution cell proliferation assay, Promega).

### H. Quantitative real time PCR

RNA was extracted from T-cells using the RNeasy Mini Kit (Qiagen). Relative quantification of EphA2-ENG mRNA expression was done using SYBR Green Reagents (Qiagen).

### I. Animal models

All animal experiments followed a protocol approved by the Baylor College of Medicine Institutional Animal Care and Use Committee. Experiments were performed as described previously with minor modifications.

*Intracranial model:* Male 8- to 12-week-old ICR-SCID mice were purchased from Taconic (IcrTac:ICR-Prkdcscid; Fox Chase C.B-17 SCID ICR; Taconic). Briefly, U373.eGFP.ffLuc cells (1x10⁵ in 2.0 µL) were injected 3 mm deep to the bregma, corresponding to the center of the right caudate nucleus over 5 minutes. Seven days after tumor cell injection, animals were treated with 2x10⁶ NT or ENG T-cells from the same donor in 2 µL to the same tumor coordinates. Photons emitted from the luciferase-expressing tumor cells were quantified using Living Image software (Caliper Life Sciences). A constant region-of-interest was drawn over the tumor region and the intensity of the signal measured as total photon/second/cm²/steradian (p/s/cm²/sr). Animals were initially imaged every two days, and once a week thereafter. Mice were euthanized when the tumor radiance was >1x10⁹ on two occasions or when they met euthanasia criteria (neurological deficits, weight loss, signs of distress) in accordance with the Center for Comparative Medicine at Baylor College of Medicine.

*Systemic A549 tumor model (antitumor activity)*: Male 8- to 12-week-old SCID Beige mice were purchased from Charles River (CB17.Cg-PrkdcscidLystbg/Crl; Fox Chase SCIDR Beige mouse; Charles River Laboratories International, Inc.). 2.5x10⁶ A549.eGFP.ffLuc cells in PBS were injected i.v. on day 0. Seven, 14 and 21 days after tumor cell injection, mice were treated i.v. with 1x10⁷ CD19-ENG T-cells or EphA2-ENG T-cells. All animals received one i.p. dose of IL2 (1,500 U) on the day of the T-cell injections. Untreated animals served as controls. Animals were imaged as described above. Mice were euthanized when they met euthanasia criteria in accordance with the Center for Comparative Medicine at Baylor College of Medicine.

*Systemic A549 tumor model (T-cell expansion and persistence)*: To determine the expansion and persistence of EphA2-ENG T-cells male 8 to 12 week old SCID Beige mice were injected i.v. with 2.5x10⁶ A549 cells in PBS on day 0. On day 7 post tumor challenge, 5 tumor-bearing mice and 5 controls were injected i.v. with an admixture of 5x10⁶ eGFP.ffLuc-expressing EphA2 ENG T-cells and 5x10⁶ NT T-cells. All mice received one i.p. dose of IL2 (1,500 U), and expansion and persistence of T-cells was monitored by serial bioluminescence imaging. To determine the expansion and persistence of bystander T-cells, male 8 to 12 week old SCID Beige mice were injected i.v. with 2.5x10⁶ A549 cells on day 0. On day 7 post tumor challenge, tumor-bearing mice were injected i.v. with an admixture of 5×10⁶ EphA2 ENG T-cells and 5×10⁶ eGFP.ffLuc-expressing T-cells or an admixture of 5x10⁶ CD19 ENG T-cells and 5x10⁶ eGFP.ffLuc-expressing T-cells (5 mice per group). All mice received one i.p. dose of IL2 (1,500 U), and expansion and persistence of T-cells was monitored by serial bioluminescence imaging.

### J. Statistical analysis

GraphPad Prism 5 software (GraphPad software, Inc.) was used for statistical analysis. Measurement data were presented as mean ± standard deviation (SD). For comparison between two groups, two-tailed t-test was used. For comparisons of three or more groups, the values were analyzed by one-way ANOVA with Bonferroni's post test. Linear regression analysis was performed to compare the antitumor activity of ENG and CAR T-cells, and activated and non-activated ENG T-cells. The significance level used was p < 0.05. For the mouse experiments, 5 mice were planned to detect a large effect size of 2, which provided at least 80% power with 5% type-I error. Although no formal randomization was carried out, a cage of 5 mice was chosen randomly. Survival, determined from the time of tumor cell injection, was analyzed by the Kaplan-Meier method and by the log-rank test.

## Claims

1. A cell comprising a polynucleotide vector encoding a bipartite molecule comprising an activation domain that binds to one or more cell surface molecules and an antigen recognition domain that binds to EphA2 and/or CD19, wherein the activation domain is a single chain variable fragment (scFv) that recognizes CD3 and wherein the cell is a T cell or NK cell.

2. The cell of claim 1, wherein the antigen recognition domain comprises scFV antibody moieties.

3. The cell of any one of claim 1 or 2, wherein the vector is a non-viral or viral vector.

4. The cell of claim 3, wherein the viral vector is selected from the group consisting of lentiviral, adenoviral, retroviral, and adeno-associated viral vector.

5. The cell of any claim 1 or 2, wherein the vector is an oncolytic vector.

6. A cell according to any one of the preceding claims for use in method of treating an individual with cancer, the method comprising the step of delivering a therapeutically effective amount of the cells to the individual.

7. The cell of claim 6 for use according to claim 6, wherein the cancer is EphA2-positive or CD19-positive.

8. The cell of claim 6 or 7 for use according to claim 6 or 7, wherein the vector is selected from the group consisting of lentiviral, adenoviral, retroviral, and adeno-associated viral vector.

9. The cell of any one of claims 6 to 8 for use according to any one of claims 6 to 8, wherein the individual is provided with an additional cancer therapy.

10. The cell of claim 9 for use according to claim 9, wherein the additional cancer therapy is surgery, radiation, chemotherapy, hormone therapy, immunotherapy, or a combination thereof.

11. The cell of any one of claims 6 to 10 for use according to any one of claims 6 to 10, wherein the cancer is breast cancer, prostate cancer, lung cancer, colon cancers, head and neck cancer, skin cancer, ovarian cancer, endometrial cancer, cervix cancer, kidney cancer, gastric cancer, cancer of the small intestine, liver cancer, pancreas cancer, gall bladder cancer, cancer of the bile duct, esophagus cancer, cancer of the salivary glands, cancer of the thyroid gland, or glioblastoma.

12. The cell of any one of claims 6 to 10 for use according to any one of claims 6 to 10, wherein the antigen recognition domain binds to CD19 and the cancer is a malignancy that expresses CD19, a haematological malignancy derived from the B-cell lineage, or a malignancy that aberrantly expresses CD19.

13. A polynucleotide vector encoding a bipartite molecule comprising an activation domain that binds to one or more cell surface molecules and an antigen recognition domain that binds to EphA2 and CD19, wherein the activation domain is a single chain variable fragment (scFv) that recognizes CD3.

14. The vector of claim 13, wherein the vector is a non-viral or viral vector.

15. The vector of claim 14, wherein the viral vector is selected from the group consisting of lentiviral, adenoviral, retroviral, and adeno-associated viral vector.

## Patentansprüche

1. Zelle, umfassend einen Polynukleotidvektor, der ein zweiteiliges Molekül kodiert, das eine Aktivierungsdomäne umfasst, die an ein oder mehrere Zelloberflächenmoleküle bindet, und eine Antigenerkennungsdomäne, die an EphA2 und/oder CD19 bindet, wobei die Aktivierungsdomäne ein scFv-Fragment (single chain variable fragment, scFv) ist, das CD3 erkennt, und wobei die Zelle eine T-Zelle oder eine NK-Zelle ist.

2. Zelle nach Anspruch 1, wobei die Antigenerkennungsdomäne scFV-Antikörperteile umfasst.

3. Zelle nach Anspruch 1 oder 2, wobei der Vektor ein nicht-viraler oder viraler Vektor ist.

4. Zelle nach Anspruch 3, wobei der virale Vektor aus der Gruppe bestehend aus einem lentiviralen, adenoviralen, retroviralen und Adeno-assoziierten viralen Vektor ausgewählt ist.

5. Zelle nach Anspruch 1 oder 2, wobei der Vektor ein onkolytischer Vektor ist.

6. Zelle nach einem der vorstehenden Ansprüche zur Verwendung in einem Verfahren zur Behandlung eines Individuums mit Krebs, wobei das Verfahren den Schritt eines Zuführens einer therapeutisch wirksamen Menge von den Zellen zu dem Individuum umfasst.

7. Zelle nach Anspruch 6 zur Verwendung nach Anspruch 6, wobei der Krebs EphA2-positiv oder CD19-positiv ist.

8. Zelle nach Anspruch 6 oder 7 zur Verwendung nach Anspruch 6 oder 7, wobei der Vektor aus der Gruppe bestehend aus einem lentiviralen, adenoviralen, retroviralen und Adeno-assoziierten viralen Vektor ausgewählt ist.

9. Zelle nach einem der Ansprüche 6 bis 8 zur Verwendung nach einem der Ansprüche 6 bis 8, wobei dem Individuum eine zusätzliche Krebstherapie bereitgestellt wird.

10. Zelle nach Anspruch 9 zur Verwendung nach Anspruch 9, wobei die zusätzliche Krebstherapie eine Operation, Bestrahlung, Chemotherapie, Hormontherapie, Immuntherapie oder eine Kombination davon ist.

11. Zelle nach einem der Ansprüche 6 bis 10 zur Verwendung nach einem der Ansprüche 6 bis 10, wobei der Krebs Brustkrebs, Prostatakrebs, Lungenkrebs, Kolonkrebs, Kopf- und Halskrebs, Hautkrebs, Ovarialkrebs, Endometriumkrebs, Cervixkrebs, Nierenkrebs, Magenkrebs, Dünndarmkrebs, Leberkrebs, Pankreaskrebs, Gallenblasenkrebs, Gallengangkrebs, Speiseröhrenkrebs, Speicheldrüsenkrebs, Schilddrüsenkrebs oder ein Glioblastom ist.

12. Zelle nach einem der Ansprüche 6 bis 10 zur Verwendung nach einem der Ansprüche 6 bis 10, wobei die Antigenerkennungsdomäne an CD19 bindet und der Krebs ein Malignom ist, das CD19 exprimiert, ein hämatologisches Malignom ist, das von B-Zellen-Abstammung abgeleitet ist oder ein Malignom ist, das CD 19 aberrant exprimiert.

13. Polynukleotidvektor, der ein zweiteiliges Molekül kodiert, umfassend eine Aktivierungsdomäne, die an ein oder mehrere Zelloberflächenmoleküle bindet, und eine Antigenerkennungsdomäne, die sich an EphA2 und CD19 bindet, wobei die Aktivierungsdomäne ein scFv-Fragment (single chain variable fragment, scFv) ist, das CD3 erkennt.

14. Vektor nach Anspruch 13, wobei der Vektor ein nicht-viraler oder viraler Vektor ist.

15. Vektor nach Anspruch 14, wobei der virale Vektor aus der Gruppe bestehend aus einem lentiviralen, adenoviralen, retroviralen und Adeno-assoziierten viralen Vektor ausgewählt ist.

## Revendications

1. Cellule comprenant un vecteur polynucléotidique codant pour une molécule bipartite comprenant un domaine d'activation qui se lie à au moins une molécule de surface cellulaire et un domaine de reconnaissance d'antigène qui se lie à EphA2 et/ou CD19, le domaine d'activation étant un fragment variable à chaîne unique (scFv) qui reconnaît CD3 et la cellule étant une cellule T ou une cellule NK.

2. Cellule selon la revendication 1, dans laquelle le domaine reconnaissance d'antigène comprend des fragments d'anticorps ScFV.

3. Cellule selon l'une quelconque des revendications 1 ou 2, dans laquelle le vecteur est un vecteur non-viral ou viral.

4. Cellule selon la revendication 3, dans laquelle le vecteur viral est choisi dans le groupe constitué par un vecteur lentiviral, adénoviral, rétroviral et viral adéno-associé.

5. Cellule selon l'une quelconque des revendications 1 ou 2, dans laquelle le vecteur est un vecteur oncolytique.

6. Cellule selon l'une quelconque des revendications précédentes destinée à une utilisation dans une méthode de traitement d'un individu atteint de cancer, la méthode comprenant l'étape consistant à administrer une quantité thérapeutiquement efficace des cellules à l'individu.

7. Cellule selon la revendication 6 destinée à une utilisation selon la revendication 6, dans laquelle le cancer est EphA2 positif ou CD-19 positif.

8. Cellule selon la revendication 6 ou 7 destinée à une utilisation selon la revendication 6 ou 7, dans laquelle le vecteur est choisi dans le groupe constitué par un vecteur lentiviral, adénoviral, rétroviral et viral adéno-associé.

9. Cellule selon l'une quelconque des revendications 6 à 8 destinée à une utilisation selon l'une quelconque des revendications 6 à 8, dans laquelle l'individu bénéficie d'une thérapie anti-cancéreuse complémentaire.

10. Cellule selon la revendication 9 destinée à une utilisation selon la revendication 9, dans laquelle la thérapie anti-cancéreuse complémentaire est la chirurgie, l'irradiation, la chimiothérapie, la thérapie hormonale, l'immunothérapie ou une combinaison de celles-ci.

11. Cellule selon l'une quelconque des revendications 6 à 10 destinée à une utilisation selon l'une quelconque des revendications 6 à 10, dans laquelle le cancer est le cancer du sein, la cancer de la prostate, le cancer du poumon, les cancers du côlon, la cancer de la tête et du cou, le cancer de la peau, le cancer ovarien, le cancer de l'endomètre, le cancer du col de l'utérus, le cancer du rein, le cancer gastrique, le cancer du petit intestin, le cancer du foie, le cancer du pancréas, le cancer de la vésicule biliaire, le cancer du canal biliaire, le cancer de l'oesophage, le cancer des glandes salivaires, le cancer de la glande thyroïde ou le glioblastome.

12. Cellule selon l'une quelconque des revendications 6 à 10 destinée à une utilisation selon l'une quelconque des revendications 6 à 10, dans laquelle le domaine de reconnaissance d'antigène se lie à CD 19 et le cancer est une tumeur maligne qui exprime CD19, une tumeur maligne hématologique dérivée de la lignée cellulaire B ou une tumeur maligne qui exprime de manière aberrante CD19.

13. Vecteur polynucléotidique codant pour une molécule bipartite comprenant un domaine d'activation qui se lie à au moins une molécule de surface cellulaire et un domaine de reconnaissance d'antigène qui se lie à EphA2 et CD19, le domaine d'activation étant un fragment variable à chaîne unique (scFv) qui reconnaît CD3.

14. Vecteur selon la revendication 13, le vecteur étant un vecteur non-viral ou viral.

15. Vecteur selon la revendication 14, le vecteur viral étant choisi dans le groupe constitué par un support lentiviral, adénoviral, rétroviral et viral adéno-associé.
